# DEMANDE DE BREVET EUROPEEN

(11) **EP 1 649 849 A2**
(43) Date de publication de la demande: **26.04.2006**
(21) Numéro de dépôt: 06000097.3
(22) Date de dépôt: 28.02.2002
(51) Int. Cl.: A61K 9/08, A61K 45/06, A61K 31/4353, A61K 31/397, A61P 25/16

(54) **Compositions pour le traitement de la maladie de parkinson contentant un antagoniste du récepteur CB1 et un produit qui active la neurotransmission dompaminerque dans le cerveau**

(30) Priorité: 29.08.2001 FR 0111200
(62) Demande divisionnaire de: 02772514.2
(71) Demandeur: Aventis Pharma S.A., 92160 Antony (FR)
(72) Inventeur: Benavides, Jesus, 92290 Chatenay Malabry (FR); Boccio, Daniel, 52500 Fayl Billot (FR); Henin, Yvette, 75019 Paris (FR); Piot-Grosjean, Odile, 94600 Choisy Le Roi (FR)
(74) Mandataire: Morel-Pécheux, Muriel

(57) **Abrégé**

La présente invention concerne l'association de un ou plusieurs dérivés d'azétidine antagonistes CB1 et de un ou plusieurs produits qui activent la neurotransmission dopaminergique dans le cerveau, les compositions pharmaceutiques les contenant et leur utilisation pour le traitement de la maladie de Parkinson.

## Description

La présence invention concerne l'association de un ou plusieurs antagonistes du récepteur CB1 et de un ou plusieurs produits qui activent la neurotransmission dopaminergique dans le cerveau, les compositions pharmaceutiques les contenant et leur utilisation pour le traitement de la maladie de Parkinson.

Des antagonistes du récepteur CB1 sont développés pour le traitement de la schizophrénie (D. KENDALL, Curr. Opin. Cent. Peripher. Nerv. Syst. Invest. Drugs, 2(1), 112-122, 2000), pour leur action sur la prise alimentaire (G. COLOMBO et coll., Life Sciences, 63 (8), 113-117 (1998); J. SIAMAND et coll., Behavioural Pharmacol., 9, 179-181 (1998)), pour le traitement de la maladie de Parkinson, épilepsie, migraine, stress (G. GERDEMAN, DM. LOVINGER, J.Neurophysiol, 85(1), 468-471, 2001 ; W00046209).

La maladie de Parkinson résulte d'un désordre neurologique chronique et progressif. Elle repose sur un déficit en dopamine, un excès relatif en acétylcholine et est associée à une destruction des neurones dopaminergiques qui participent au contrôle des activités motrices (H. LULLMANN et coll., Atlas de poche de pharmacologie, 2° Ed, Médecine-Sciences, Flammarion, ISBN2-257-12119-8). Le traitement de la maladie de Parkinson est principalement pharmacologique et fait appel à différents médicaments destinés à accroître la quantité de dopamine présente dans le cerveau.

La dopamine ne traversant pas la barrière hémato-encéphalique, la lévodopa, précurseur de la dopamine convertie en dopamine par la dopa-décarboxylase, a été développée dans les années 60. La lévodopa reste aujourd'hui le premier traitement de choix de la maladie de Parkinson et donne initialement de bons résultats mais après plusieurs années, on observe chez la majorité des patients des fluctuations de réponse (effet 'on-off'), une diminution de son efficacité au fur et à mesure que la maladie progresse (effet 'wearing-off', détérioration de fin de dose), et surtout des dyskinésies (mouvements anormaux involontaires). Un état de psychose peut également être observé.

D'autres médicaments comme les agonistes dopaminergiques sont également préconisés seuls ou en association à la lévodopa et ont principalement pour objet de réduire au minimum les effets indésirables de celle-ci. Depuis quelques années, des inhibiteurs sélectifs de la monoamine oxydase MAO-B, enzyme de dégradation de la dopamine dans le cerveau, ainsi que des inhibiteurs de la catéchol-O méthyl-transférase (COMT), enzyme qui empêche la lévodopa de franchir la barrière hémato-encéphalique, ont été développés et prescrits en association avec la lévodopa. Des effets secondaires importants ont également été observés avec ces thérapies.

Afin de remédier aux inconvénients susmentionnés, il a été trouvé que l'association de un ou plusieurs antagonistes du récepteur CB 1 et de un ou plusieurs produits qui activent la neurotransmission dopaminergique dans le cerveau présente un effet de synergie dans le traitement de la maladie de Parkinson. En effet cette association permettrait de potentialiser les effets symptomatiques d'une monothérapie dopaminergique (lévodopa, agonistes dopaminergiques et inhibiteurs d'enzyme) et permettrait de réduire les effets secondaires, en particulier les dyskinésies.

Outre la lévodopa, précurseur de la dopamine, on peut citer parmi les agonistes dopaminergiques, les produits suivants : bromocriptine (Novartis), cabergoline (Pharmacia Corp.) adrogolide (Abbott Laboratories), BAM-1110(Maruko Seiyaku Co Ltd), Duodopa® (Neopharma), L-dopa, dopadose (Neopharma), CHF1512 (Chiesi), NeuroCell-PD (Diacrin Inc), PNU-95666 (Pharmacia & Upjohn), ropinirole (GlaxoSmithKline Beecham), pramipexole (Boehringer Ingelheim) rotigotine (Discovery Therapeutics, Lohmann Therapie System), spheramine (Titan Pharmaceuticals), TV 1203 (Teva pharmaceutical), uridine (Polifarma).

Parmi les inhibiteurs de MAO_{B}, on peut citer: rasagiline (Teva Pharmaceutical Ind.) selegiline (RPScherer Corp / Elan) SL340026 (Sanofi-Synthelabo).

Parmi les inhibiteurs de COMT, on peut citer: tolcapone (Roche) et entacapone (Orion Pharma).

L'invention a donc pour objet l'association de un ou plusieurs produits activant la neurotransmission dopaminergique dans le cerveau et de un ou plusieurs dérivés d'azétidine antagonistes CB1 de formule I :

Parmi les antagonistes CB1, on peut notamment utiliser les dérivés d'azétidine décrits dans les demandes de brevet FR 0002775, FR0002777, FR 0002776 de formule I. 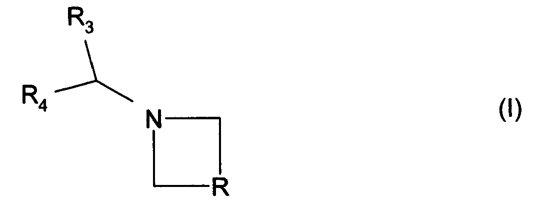 dans laquelle
**soit A :**
R représente un radical CR₁R₂, C=C(R₅)SO₂R₆ ou C=C(R₇)SO₂alk,
soit R₁ représente un atome d'hydrogène et R₂ représente un radical -C(R₈)(R₉)(R₁₀), -C(R₈)(R₁₁)(R₁₂), -CO-NR₁₃R₁₄, -CH₂-CO-NR₁₃R₁₄, -CH₂-CO-R₆, -CO-R₆, -CO-cycloalkyle, -SO-R₆, -SO₂-R₆, -C(OH)(R₁₂)(R₆), -C(OH)(R₆)(alkyle), -C(=NOalk)R₆, -C(=NO-CH₂-CH=CH₂)R₆, -CH₂-CH(R₆)NR₃₁R₃₂, -CH₂-C(=NOalk)R₆, -CH(R₆)NR₃₁R₃₂, -CH(R₆)NHSO₂alk, -CH(R₆)NHCONHalk ou -CH(R₆)NHCOalk,
soit R₁ représente un radical alkyle, NH-R₁₅, cyano, -S-alk-NR₁₆R₁₇, -CH₂-NR₁₈R₁₉, ou -NR₂₀R₂₁ et R₂ représente un radical -C(R₈)(R₁₁)(R₁₂),
R₃ et R₄, identiques ou différents, représentent soit un radical alkyle ou cycloalkyle, soit un aromatique choisi parmi phényle, naphtyle ou indényle, ces aromatiques étant non substitués ou substitués par un ou plusieurs halogène, alkyle, alcoxy, formyle, hydroxy, trifluorométhyle, trifluorométhoxy, -CO-alk, cyano, -COOH, -COOalk, -CONR₂₂R₂₃, -CO-NH-NR₂₄R₂₅, alkylsulfanyle, alkylsulfinyle, alkylsulfonyle, alkylsulfanylalkyle, alkylsulfinylalkyle, alkylsulfonylalkyle, hydroxyalkyle ou -alk-NR₂₄R₂₅; soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, furyle, imidazolyle, isochromannyle, isoquinolyle, pyrrolyle, pyridyle, pyrimidinyle, quinolyle, 1,2,3,4-tétrahydroisoquinolyle, thiazolyle, thiényle, ces hétéroaromatiques pouvant être non substitués ou substitués par un ou plusieurs halogène, alkyle, alcoxy, hydroxy, trifluorométhyle, trifluorométhoxy, cyano, -COOH, -COOalk, -CO-NH-NR₂₄R₂₅, -CONR₂₂R₂₃, -alk-NR₂₄R₂₅, alkylsulfanyle, alkylsulfinyle, alkylsulfonyle, alkylsulfanylalkyle, alkylsulfinylalkyle, alkylsulfonylalkyle ou hydroxyalkyle,
R₅ représente un atome d'hydrogène ou un radical alkyle,
R₆ représente un radical Ar₁ ou Het₁,
R₇ représente un radical cycloalkyle, hétérocycloalkyle ou hétérocyclényle éventuellement substitué par un radical -CSO-phényle,
R₈ représente un atome d'hydrogène ou un radical alkyle,
R₉ représente un radical -CO-NR₂₆R₂₇, -COOH, -COOalk, -CH₂OH, -NH-CO-NH-alk, -CH₂-NHR₂₈ ou -NHCOOalk,
R₁₀ représente un radical Ar₁ ou Het₁,
R₁₁ représente un radical -SO₂-alk, -SO₂-Ar₁, -SO₂-Het₁,
R₁₂ représente un atome d'hydrogène ou un radical Ar₁ ou Het₁,
R₁₃ représente un atome d'hydrogène ou un radical alkyle,
R₁₄ représente un radical Ar₁, Het₁, -alk-Ar₁ ou -alk-Het₁,
R₁₅ représente un radical alkyle, cycloalkyle ou -alk-NR₂₉R₃₀,
R₁₆ et R₁₇, identiques ou différents, représentent un atome d'hydrogène ou un radical alkyle ou bien R₁₆ et R₁₇ forment ensemble avec l'atome d'azote auquel ils sont rattachés un hétérocycle mono ou bicyclique insaturé ou saturé, ayant 3 à 10 chaînons et contenant éventuellement un ou plusieurs autres hétéroatomes choisis parmi oxygène, soufre et azote et éventuellement substitué par un ou plusieurs radicaux alkyle,
R₁₈ représente un atome d'hydrogène ou un radical alkyle,
R₁₉ représente un atome d'hydrogène ou un radical alkyle, cycloalkyle, cycloalkylalkyle, cycloalkylcarbonyle, -SO₂alk, -CO-NHalk ou -COOalk,
ou bien R₁₈ et R₁₉ forment avec l'atome d'azote auquel ils sont rattachés un hétérocycle mono ou bicyclique insaturé ou saturé, ayant 3 à 10 chaînons et contenant éventuellement un ou plusieurs hétéroatomes choisis parmi oxygène, soufre et azote et éventuellement substitué par un ou plusieurs radicaux alkyle,
-NR₂₀R₂₁ représente un hétérocycle monocyclique saturé ou insaturé ayant 3 à 8 chaînons et contenant éventuellement un autre hétéroatome choisi parmi oxygène, azote et soufre,
R₂₂ et R₂₃, identiques ou différents, représentent un atome d'hydrogène ou un radical alkyle ou bien R₂₂ et R₂₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
R₂₄ et R₂₅, identiques ou différents, représentent un atome d'hydrogène ou un radical alkyle, -COOalk, cycloalkyle, alkylcycloalkyle, -alk-O-alk, hydroxyalkyle ou bien R₂₄ et R₂₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, -COalk, -COOalk, -CO-NHalk, -CS-NHalk, oxo, hydroxyalkyle, -alk-O-alk, -CO-NH₂,
R₂₆ et R₂₇, identiques ou différents, représentent un atome d'hydrogène ou un radical alkyle, hydroxyalkyle, cycloalkyle, cycloalkylalkyle, -alk-COOalk, -alk-Ar₁, -alk-Het₁, Het₁, -alk-N(alk)₂, R₂₆ et R₂₇ peuvent également former avec l'atome d'azote auquel ils sont rattachés un hétérocycle mono ou bicyclique insaturé ou saturé, ayant 3 à 10 chaînons et contenant éventuellement un ou plusieurs autres hétéroatomes choisis parmi oxygène, soufre et azote et éventuellement substitué par un ou plusieurs radicaux alkyle, alcoxy, halogène,
R₂₈ représente un radical -CH₂-alk, benzyle, -SO₂alk, -CONHalk, -COalk, cycloalkylalkylcarbonyle, cycloalkylcarbonyle, -CO-(CH₂)ₙOH,
n est égal à 1, 2 ou 3,
R₂₉ et R₃₀, identiques ou différents, représentent un atome d'hydrogène ou un radical alkyle ou bien R₂₉ et R₃₀ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
   R₃₁ et R₃₂, identiques ou différents, représentent un atome d'hydrogène ou un radical alkyle, Ar₁ ou -alk-Ar₁ ou bien R₃₁ et R₃₂ forment ensemble avec l'atome d'azote auquel ils sont rattachés un hétérocycle choisi parmi aziridinyle, azétidinyle, pyrrolidinyle et pipéridinyle,
Ar₁ représente un radical phényle ou naphtyle éventuellement substitué par un ou plusieurs substituants choisis parmi halogène, alkyle, alcoxy, -CO-alk, cyano, -COOH, -COOalk, -CONR₂₂R₂₃, -CO-NH-NR₂₄R₂₅, alkylsulfanyle, alkylsulfinyle, alkylsulfonyle, alkylsulfanylalkyle, alkylsulfinylalkyle, alkylsulfonylalkyle, hydroxyalkyle, -alk-NR₂₄R₂₅, -NR₂₄R₂₅, alkylthioalkyle, formyle, hydroxy, CF₃, OCF₃, Het₁, -O-alk-NH-cycloalkyle ou SO₂NH₂,
Het₁ représente un hétérocycle mono ou bicyclique insaturé ou saturé, ayant 3 à 10 chaînons et contenant un ou plusieurs hétéroatomes choisis parmi oxygène, soufre et azote et éventuellement substitué par un ou plusieurs halogène, alkyle, alcoxy, alcoxycarbonyle, -CONR₂₂R₂₃, hydroxy, hydroxyalkyle, oxo ou SO₂NH₂,
**soit B :**
R représente un radical CHR₃₃
R₃₃ représente un radical -NHCOR₃₄ ou -N(R₃₅)-Y-R₃₆,
Y est CO ou SO₂,
R₃ et R₄, identiques ou différents, représentent soit un aromatique choisi parmi phényle, naphtyle et indényle, ces aromatiques étant non substitués ou substitués par un ou plusieurs halogène, alkyle, alcoxy, formyle, hydroxy, trifluorométhyle, trifluorométhoxy, -CO-alk, cyano, -COOH, -COOalk, -CONR₃₇R₃₈, -CO-NH-NR₃₉R₄ₒ, alkylsulfanyle, alkylsulfinyle, alkylsulfonyle, alkylsulfanylalkyle, alkylsulfinylalkyle, alkylsulfonylalkyle, hydroxyalkyle, ou -alk-NR₃₇R₃₈; soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, pyrimidinyle, furyle, imidazolyle, isochromannyle, isoquinolyle, pyrrolyle, pyridyle, quinolyle, 1,2,3,4-tétrahydroisoquinolyle, thiazolyle et thiényle, ces hétéroaromatiques pouvant être non substitués ou substitués par un halogène, alkyle, alcoxy, hydroxy, trifluorométhyle, trifluorométhoxy, cyano, -COOH, -COOalk, -CO-NH-NR₃₉R₄₀, -CONR₃₇R₃₈, -alk-NR₃₉R₄₀, alkylsulfanyle, alkylsulfinyle, alkylsulfonyle, alkylsulfanylalkyle, alkylsulfinylalkyle, alkylsulfonylalkyle ou hydroxyalkyle,
R₃₄ représente un radical -alk-SO₂-R₄₁, -alk-SO₂-CH=CH-R₄₁, Het₂ substitué par - SO₂-R₄₁ ou phényle substitué par -SO₂-R₄₁ ou -alk-SO₂-R₄₁,
R₃₅ représente un atome d'hydrogène ou un radical alkyle,
R₃₆ représente un radical phénylalkyle, Het₂ ou Ar₂,
R₃₇ et R₃₈, identiques ou différents, représentent un atome d'hydrogène ou un radical alkyle ou bien R₃₇ et R₃₈ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₃₉ et R₄₀, identiques ou différents représentent un atome d'hydrogène ou un radical alkyle, -COOalk, cycloalkyle, alkylcycloalkyle, -alk-O-alk ou hydroxyalkyle ou bien R₃₉ et R₄₀ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, -COalk, -COOalk, -CO-NHalk, -CS-NHalk, oxo, hydroxyalkyle, -alk-O-alk ou -CO-NH₂,
R₄₁ représente un radical alkyle, Ar₂ ou Het₂,
Ar₂ représente un radical phényle, naphtyle ou indènyle, ces radicaux étant éventuellement substitués par un ou plusieurs halogène, alkyle, alcoxy, cyano, -CO-alk, -COOH, -COOalk, -CONR₄₂R₄₃, -CO-NH-NR₄₄R₄₅, alkylsulfanyle, alkylsulfinyle, alkylsulfonyle, -alk-NR₄₄R₄₅, -NR₄₄R₄₅, alkylthioalkyle, formyle, hydroxy, hydroxyalkyle, Het₂, -O-alk-NH-cycloalkyle, OCF₃, CF₃, -NH-CO-alk, -SO₂NH₂, -NH-COCH₃, -NH-COOalk, Het₂ ou bien sur 2 atomes de carbone adjacents par un dioxyméthylène,
Het₂ représente un hétérocycle mono ou bicyclique insaturé ou saturé, ayant 3 à 10 chaînons et contenant un ou plusieurs hétéroatomes choisis parmi oxygène, soufre et azote éventuellement substitué par un ou plusieurs alkyle, alcoxy, vinyle, halogène, alcoxycarbonyle, oxo, hydroxy, OCF₃ ou CF₃, les hétérocycles azotés étant éventuellement sous leur forme N-oxydée,
R₄₂ et R₄₃, identiques ou différents, représentent un atome d'hydrogène ou un radical alkyle ou bien R₄₂ et R₄₃ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs radicaux alkyle,
R₄₄ et R₄₅, identiques ou différents, représentent un atome d'hydrogène ou un radical alkyle, -COOalk, cycloalkyle, alkylcycloalkyle, -alk-O-alk, hydroxyalkyle ou bien R₄₄ et R₄₅ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs radicaux alkyle, -COalk, -COOalk, -CO-NHalk, -CS-NHalk, oxo, hydroxyalkyle, -alk-O-alk, -CO-NH₂,
**soit C :**
R représente un radical CHR₄₆
R₄₆ représente un radical -N(R₄₇)R₄₈, -N(R₄₇)-CO-R₄₈, -N(R₄₇)-SO₂R₄₉,
R₃ et R₄, identiques ou différents, représentent soit un aromatique choisi parmi phényle, naphtyle et indényle, ces aromatiques étant non substitués ou substitués par un ou plusieurs halogène, alkyle, alcoxy, formyle, hydroxy, trifluorométhyle, trifluorométhoxy, -CO-alk, cyano, -COOH, COOalk, -CONR₅₀R₅₁, -CO-NH-NR₅₂R₅₃, alkylsulfanyle, alkylsulfinyle, alkylsulfonyle, alkylsulfanylalkyle, alkylsulfinylalkyle, alkylsulfonylalkyle, hydroxyalkyle ou -alk-NR₇R₈; soit un hétéroaromatique choisi parmi les cycles benzofuryle, benzothiazolyle, benzothiényle, benzoxazolyle, chromannyle, 2,3-dihydrobenzofuryle, 2,3-dihydrobenzothiényle, furyle, imidazolyle, isochromannyle, isoquinolyle, pyrrolyle, pyridyle, pyrimidyle, quinolyle, 1,2,3,4-tétrahydroisoquinolyle, thiazolyle et thiényle, ces hétéroaromatiques pouvant être non substitués ou substitués par un halogène, alkyle, alcoxy, hydroxy, trifluorométhyle, trifluorométhoxy, cyano, -COOH, COOalk, -CO-NH-NR₅₂R₅₃, -CONR₅₀R₅₁, -alk-NR₅₂R₅₃, alkylsulfanyle, alkylsulfinyle, alkylsulfonyle, alkylsulfanylalkyle, alkylsulfinylalkyle, alkylsulfonylalkyle ou hydroxyalkyle ,
R₄₇ représente un radical -C(R₅₄)(R₅₅)-Het₃, -Het₃, -C(R₅₄)(R₅₅)-Ar₃, Ar₃, cycloalkyle ou norbomyle,
R₄₈ représente un atome d'hydrogène ou un radical hydroxyalkyle, -alk-COOalk, -alk-CONR₅₀R₅₁, -alk-NR₅₀R₅₁, alcoxy, Ar₃, Het₃, -CH₂Ar₃, -CH₂Het₃ ou alkyle éventuellement substitué par un ou plusieurs halogène,
R₄₉ représente un radical hydroxyalkyle, -alk-COOalk, -alk-CONR₅₀R₅₁, -alk-NR₅₀R₅₁, alcoxy, Ar₃, Het₃, -CH₂Ar₃, -CH₂Het₃ ou alkyle éventuellement substitué par 1 ou plusieurs halogène,
R₅₀ et R₅₁, identiques ou différents, représentent un atome d'hydrogène ou un radical alkyle ou bien R₅₀ et R₅₁ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₅₂ et R₅₃, identiques ou différents, représentent un atome d'hydrogène ou un radical alkyle, -COOalk, cycloalkyle, alkylcycloalkyle, -alk-O-alk, hydroxyalkyle ou bien R₅₂ et R₅₃ forment ensemble avec l'atome d'azote auquel ils sont rattachés un hétérocycle mono ou bicyclique saturé ou insaturé ayant 3 à 10 chaînons, contenant éventuellement un autre hétéroatome choisi parmi oxygène, soufre et azote et étant éventuellement substitué par un ou plusieurs alkyle, -COalk, -COOalk, -CO-NHalk, -CS-NHalk, oxo, hydroxyalkyle, -alk-O-alk ou -CO-NH₂,
R₅₄ représente un atome d'hydrogène ou un radical hydroxyalkyle, -alk-COOalk, -alk-CONR₅₀R₅₁, -alk-NR₅₀R₅₁, alcoxyalkyle, Ar₃, Het₃, -CH₂Ar₃, -CH₂Het₃ ou alkyle éventuellement substitué par un ou plusieurs halogène,
R₅₅ représente un atome d'hydrogène ou un radical hydroxyalkyle, -alk-COOalk, -alk-CONR₅₀R₅₁, -alk-NR₅₀R₅₁, alcoxyalkyle ou alkyle éventuellement substitué par un ou plusieurs halogène,
ou bien R₅₄ et R₅₅ forment ensemble avec l'atome de carbone auquel ils sont rattachés un cycle mono ou bicyclique saturé ayant 3 à 10 chaînons, contenant éventuellement un autre hétéroatome choisi parmi oxygène, soufre et azote et étant éventuellement substitué par un ou plusieurs alkyle,
Ar₃ représente un radical phényle, naphtyle ou indènyle, ces différents radicaux étant éventuellement substitués par un ou plusieurs halogène, alkyle, alcoxy, -CO-alk, cyano, -COOH, -COOalk, -CONR₅₆R₅₇, -CO-NH-NR₅₈R₅₉, alkylsulfanyle, alkylsulfinyle, alkylsulfonyle, -alk-NR₅₈R₅₉, -NR₅₈R₅₉, alkylthioalkyle, formyle, CF₃, OCF₃, Het₃, -O-alk-NH-cycloalkyle, SO₂NH₂, hydroxy, hydroxyalkyle, -NHCOalk, NHCOOalk ou sur 2 atomes de carbone adjacents par dioxyméthylène,
Het₃ représente un hétérocycle mono ou bicyclique insaturé ou saturé, ayant 3 à 10 chaînons et contenant un ou plusieurs hétéroatomes choisi parmi oxygène, soufre et azote éventuellement substitué par un ou plusieurs alkyle, alcoxy, halogène, alcoxycarbonyle, oxo, hydroxy, les hétérocycles azotés étant éventuellement sous leur forme N-oxydée,
R₅₆ et R₅₇, identiques ou différents, représentent un atome d'hydrogène ou un radical alkyle ou bien R₅₆ et R₅₇ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
R₅₈ et R₅₉, identiques ou différents, représentent un atome d'hydrogène ou un radical alkyle ou bien R₅₈ et R₅₉ forment ensemble avec l'atome d'azote auquel ils sont rattachés un hétérocycle mono ou bicyclique saturé ayant 3 à 10 chaînons, contenant éventuellement un autre hétéroatome choisi parmi oxygène, soufre et azote et étant éventuellement substitué par un ou plusieurs alkyle,
alk représente un radical alkyle ou alkylène,
   les radicaux et portions alkyle et alkylène et les radicaux et portions alcoxy sont en chaîne droite ou ramifiée et contiennent 1 à 6 atomes de carbone, les radicaux cycloalkyle contiennent 3 à 10 atomes de carbone et les radicaux hétérocycloalkyle et hétérocyclényle contiennent 3 à 10 atomes de carbone,
les isomères optiques de ces composés et leurs sels avec un acide minéral ou organique pharmaceutiquement acceptables.

Parmi les dérivés d'azétidine préférés objet de la présente invention on peut citer les dérivés suivants :
(RS)-1-[bis(4-chlorophényl)méthyl)]-3-[(3,5-difluorophényl)(méthylsulfonyl) méthyl] -azétidine,
(R)-1-[bis(4-chlorophényl)méthyl)]-3-[(3,5-difluorophényl)(méthylsulfonyl) méthyl]-azétidine,
(S)-1-[bis(4-chlorophényl)méthyl)]-3-[(3,5-difluorophényl)(méthylsulfonyl) méthyl]-azétidine,
(RS)-1-[bis(4-chlorophényl)méthyl)]-3-[(pyrid-3-yl)(méthylsulfonyl)méthyl]-azétidine,
(R)-1-[bis(4-chlorophényl)méthyl)]-3-[(pyrid-3-yl)(méthylsulfonyl)méthyl]-azétidine,
(S)-1-[bis(4-chlorophényl)méthyl)]-3-[(pyrid-3-yl)(méthylsulfonyl)méthyl]-azétidine,
(RS)-1-[bis-(3-fluorophényl)méthyl]-3-[(3,5-difluorophényl)méthylsulfonyl-méthyl]azétidine,
(R)-1-[bis-(3-fluorophényl)méthyl]-3-[(3,5-difluorophényl)méthylsulfonyl-méthyl]azétidine,
(S)-1-[bis-(3-fluorophényl)méthyl]-3-[(3,5-difluorophényl)méthylsulfonyl-méthyl]azétidine,
1-[bis-(4-chlorophényl)méthyl]-3-(RS)-{[3-azétidin-1-yl-phényl]méthylsulfonyl-méthyl}azétidine,
1-[bis-(4-chlorophényl)méthyl]-3-(R)-{[3-azétidin-1-yl-phényl]méthylsulfonyl-méthyl}azétidine,
1-[bis-(4-chlorophényl)méthyl]-3-(S)-{[3-azétidin-1-yl-phényl]méthylsulfonyl-méthyl}azétidine,
(RS)-1-[3-({1-[bis-(4-chlorophényl)méthyl]azétidin-3-yl}méthylsulfonyl-méthyl)phényl]pyrrolidine,
(R)-1-[3-({1-[bis-(4-chlorophényl)méthyl]azétidin-3-yl)méthylsulfonyl-méthyl)phényl]pyrrolidine,
(S)-1-[3-({1-[bis-(4-chlorophényl)méthyl]azétidin-3-yl}méthylsulfonyl-méthyl)phényl]pyrrolidine,
(RS)-N-[3-({1-[bis-(4-chlorophényl)méthyl]azétidin-3-yl}méthylsulfonyl-méthyl)phényl]-N-méthyl-amine,
(R)-N-[3-( {1 -[bis-(4-chlorophényl)méthyl]azétidin-3-yl}méthylsulfbnyl-méthyl)phényl]-N-méthyl-amine,
(S)-N-[3-({1-[bis-(4-chlorophényl)méthyl]azétidin-3-yl}méthylsulfonyl-méthyl)phényl]-N-méthyl-amine,
(RS)-1-[bis-(4-chlorophényl)méthyl]-3-[(3,5-bis-trifluorométhylphényl) méthylsulfonyl-méthyl]azétidine,
(R)-1-[bis-(4-chlorophényl)méthyl]-3-[(3,5-bis-trifluorométhylphényl) méthylsulfonyl-méthyl]azétidine,
(S)-1-[bis-(4-chlorophényl)méthyl]-3-[(3,5-bis-trifluorométhylphényl) méthylsulfonyl-méthyl]azétidine
1-[bis-(4-chlorophényl)méthyl]-3-(phénylsulfonylméthyl)-azétidine,
(RS)-1-[bis-(4-chlorophényl)-méthyl]-3-[(3,5-difluorophényl)-méthylsulfonyl-méthyl]-3-méthyl-azétidine,
(R)-1-[bis-(4-chlorophényl)-méthyl]-3-[(3,5-difluorophényl)-méthylsulfonyl-méthyl]-3-méthyl-azétidine,
(S)-1-[bis-(4-chlorophényl)-méthyl]-3-[(3,5-difluorophényl)-méthylsulfonyl-méthyl]-3-méthyl-azétidine,
(RS)-2-{1-[bis-(4-chlorophényl)méthyl]-azétidin-3-yl}-2-(3,5-difluorophényl)-N-cyclohexylacétamide,
(R)-2-{1-[bis-(4-chlorophényl)méthyl]-azétidin-3-yl}-2-(3,5-difluorophényl)-N-cyclohexylacétamide,
(S)-2-{1-[bis-(4-chlorophényl)méthyl]-azétidin-3-yl}-2-(3,5-difluorophényl)-N-cyclohexylacétamide,
(RS)-2-{1-[bis-(4-chlorophényl)méthyl]-azétidin-3-yl}-2-(3,5-difluorophényl)-N-isobutylacétamide,
(R)-2- {1-[bis-(4-chlorophényl)méthyl]-azétidin-3-yl}-2-(3,5-difluorophényl)-N-isobutylacétamide,
(S)-2- {1-[bis-(4-chlorophényl)méthyl]-azétidin-3-yl}-2-(3,5-difluorophényl)-N-isobutylacétamide,
(RS)-2- {1-[bis-(4-chlorophényl)méthyl]-azétidin-3-yl}-2-(3,5-difluorophényl)-N-cyclopropylméthyl acétamide,
(R)-2- {1-[bis-(4-chlorophényl)méthyl]-azétidin-3-yl}-2-(3,5-difluorophényl)-N-cyclopropylméthyl acétamide,
(S)-2-{1-[bis-(4-chlorophényl)méthyl]-azétidin-3-yl}-2-(3,5-difluorophényl)-N-cyclopropylméthyl acétamide,
(RS)-2-{1-[bis-(4-ehlorophényl)méthyl]-azétidin-3-yl}-2-(3,5-difluorophényl)-N-isopropylacétamide,
(R)-2-{1-[bis-(4-chlorophényl)méthyl]-azétidin-3-yl}-2-(3,5-difluorophényl)-N-isopropylacétamide,
(S)-2-{1-[bis-(4-chlorophényl)méthyl]-azétidin-3-yl}-2-(3,5-difluorophényl)-N-isopropylacétamide,
(RS)-1-[bis-(4-chlorophényl)-méthyl]-3-[1-(3,5-difluorophényl)-1-méthylsulfonyl-éthyl]-azétidine,
(R)-1-[bis-(4-chlorophényl)-méthyl]-3-[1-(3,5-difluorophényl)-1-méthylsulfonyl-éthyl]-azétidine,
(S)-1-[bis-(4-chlorophényl)-méthyl]-3-[1-(3,5-difluorophényl)-1-méthylsulfonyl-éthyl]-azétidine,
(RS)-1-[bis-(4-fluoro-phényl)-méthyl]-3-[(3,5-difluorophényl)-méthylsulfonyl-méthyl]-azétidine,
(R)-1-[bis-(4-fluoro-phényl)-méthyl]-3-[(3,5-difluorophényl)-méthylsulfonyl-méthyl]-azétidine,
(S)-1-[bis-(4-fluoro-phényl)-méthyl]-3-[(3,5-difluorophényl)-méthylsulfonyl-méthyl]-azétidine,
(RS)-{1-[(3-pyridyl)-(4-chlorophényl)méthyl]-3-[(3,5-difluorophényl)-méthylsulfonyl-méthyl]-azétidine,
(SS)-{1-[(3-pyridyl)-(4-chlorophényl)méthyl]-3-[(3,5-difluorophényl)-méthylsulfonyl-méthyl]-azétidine,
(RR)-{1-[(3-pyridyl)-(4-chlorophényl)méthyl]-3-[(3,5-difluorophényl)-méthylsulfonyl-méthyl]-azétidine,
(SR)-{1-[(3-pyridyl)-(4-chlorophényl)méthyl]-3-[(3,5-difluorophényl)-méthylsulfonyl-méthyl]-azétidine,
(RS)-{1-[(4-pyridyl)-(4-chlorophényl)méthyl]-3-[(3,5-difluorophényl)-méthylsulfonyl-méthyl]-azétidine,
(SS)-{1-[(4-pyridyl)-(4-chlorophényl)méthyl]-3-[(3,5-difluorophényl)-méthylsulfonyl-méthyl]-azétidine,
(RR)-{1-[(4-pyridyl)-(4-chlorophényl)méthyl]-3-[(3,5-difluorophényl)-méthylsulfonyl-méthyl]-azétidine,
(SR)-{1-[(4-pyridyl)-(4-chlorophényl)méthyl]-3-[(3,5-difluorophényl)-méthylsulfonyl-méthyl]-azétidine,
(RS)-5-((4-chlorophényl)-{3-[(3,5-difluorophényl)-méthylsulfonyl-méthyl]-azétidin-1-yl}-méthyl)-pyrimidine,
(SR)-5-((4-chlorophényl)-{3-[(3,5-difluorophényl)-méthylsulfonyl-méthyl]-azétidin-1-yl}-méthyl)-pyrimidine,
(RR)-5-((4-chlorophényl)-{3-[(3,5-difluorophényl)-méthylsulfonyl-méthyl]-azétidin-1-yl}-méthyl)-pyrimidine,
(SS)-5-((4-chlorophényl)-{3-[(3,5-difluorophényl)-méthylsulfonyl-méthyl]-azétidin-1-yl}-méthyl)-pyrimidine,
(SS)-{1-[(2-chloro-pyrid-5-yl)-(4-chlorophényl)méthyl]-3-[(3,5-difluorophényl)-méthylsulfonyl-méthyl]-azétidine,
(RR)-{1-[(2-chloro-pyrid-5-yl)-(4-chlorophényl)méthyl]-3-[(3,5-difluorophényl)-méthylsulfonyl-méthyl]-azétidine,
(RS{1-[(2-chloro-pyrid-5-yl)-(4-chlorophényl)méthyl]-3-[(3,5-difluorophényl)-méthylsulfonyl-méthyl]-azétidine,
(SR)-{1-[(2-chloro-pyrid-5-yl)-(4-chlorophényl)méthyl]-3-[(3,5-difluorophényl)-méthylsulfonyl-méthyl]-azétidine,
N-{1-[bis(4-chlorophényl)méthyl]azétidin-3-yl}-thièn-2-yl-sulfonamide,
N-{1-[bis(4-chlorophényl)méthyl]azétidin-3-yl}-4-méthoxyphénylsulfonamide,
N-[4-(N-{1-[bis(4-chlorophényl)méthyl]azétidin-3-yl}sulfamoyl)phényl]acétamide,
N-{1-[bis(4-chlorophényl)méthyl]azétidin-3-yl}-4-méthylphénylsulfonamide,
N-{1-[bis(4-chlorophényl)méthyl]azétidin-3-yl}-3,4-diméthoxyphénylsulfonamide,
N-{1-[bis(4-chlorophényl)méthyl]azétidin-3-yl}-3-fluorophénylsulfonamide,
N-{1-[bis(4-chlorophényl)méthyl]azétidin-3-yl}-3,4-dichlorophénylsulfonamide,
N-{1-[bis(4-chlorophényl)méthyl]azétidin-3-yl}-3-cyanophénylsulfonamide,
N-{1-[bis(4-chlorophényl)méthyl]azétidin-3-yl}-2,5-diméthoxyphénylsulfonamide,
N-{1-[bis(4-chlorophényl)méthyl]azétidin-3-yl}-3-trifluorométhylphénylsulfonamide,
N-{1-[bis(4-chlorophényl)méthyl]azétidin-3-yl}-napht-2-yl-sulfonamide,
N-{1-[bis(4-chlorophényl)méthyl]azétidin-3-yl}napht-1-yl-sulfonamide,
N-{1-[bis(4-chlorophényl)méthyl]azétidin-3-yl]}-3,4-difluorophénylsulfonamide,
N-{1-[bis(4-chlorophényl)méthyl]azétidin-3-yl}-1-méthyl-1-*H*-imidazol-4-yl-sulfonamide,
N-[4-(N- {1-[bis(4-chlorophényl)méthyl]azétidin-3-yl} sulfamoyl)-2-chlorophényl]acétamide,
N-{1-[bis(4-chlorophényl)méthyllazétidin-3-yl)pyrid-3-yl-sulfonamide,
N-{1-[bis(4-chlorophényl)méthyl]azétidin-3-yl}-4-fluorophénylsulfonamide,
N-{1-[bis(4-chlorophényl)méthyl]azétidin-3-yl}quinol-8-ylsulfonamide,
N-{1-[bis(4-chlorophényl)méthyl]azétidin-3-yl}phénylsulfonamide,
N-{1-[bis(4-chlorophényl)méthyl]azétidin-3-yl}-(phénylméthyl)sulfonamide,
N-{1-[bis(4-chlorophényl)méthyl]azétidin-3-yl}-3,5-difluorophénylsulfonamide,
N-{1-[bis(4-chlorophényl)méthyl]azétidm-3-yl}pyrid-2-ylsulfonamide,
N-{1-[bis(4-chlorophényl)méthyl]azétidin-3-yl}-(3-fluoro-5-pyrrolidin-1-yl-phényl)sulfonamide,
N-{1-[bis(4-chlorophényl)méthyl]azétidin-3-yl}-N-méthyl--4-fluorophénylsulfonamide,
N-{1-[bis(4-chlorophényl)méthyl]azétidin-3-yl}-N-méthyl-quinol-8-ylsulfonamide,
N-{1-[bis(4-chlorophényl)méthyl]azétidin-3-yl}-N-méthyl-phénylsulfonamide,
N-{1-[bis(4-chlorophényl)méthyl]azétidin-3-yl}-N-méthyl-(phénylméthyl)sulfonamide,
N-{1-[bis(4-chlorophényl)méthyl]azétidin-3-yl}-3-sulfamoylphénylsulfonamide,
2-benzènesulfonyl-N- {1-[bis-(4-chlorophényl)-méthyl]-azétidin-3-yl}-acétamide,
N-{1-[bis-(4-chlorophényl)-méthyl]-azétidin-3-yl}-2-(toluène-4-sulfonyl)-acétamide,
(3-chloro-4-méthylsulfonyl-thiophène-2-carboxy)- {1-[bis-(4-chlorophényl)-méthyl]-azétidin-3-yl}-amide,
N-{1-[bis-(4-chlorophényl)-méthyl]-azétidin-3-yl}-3-(2-phényl-éthylènesulfonyl)-propionamide,
N-{1-[bis-(4-chlorophényl)-méthyl]-azétidin-3-yl}-4-méthylsulfonyl-benzamide,
(5-méthylsulfonyl-thiophène-2-carboxy)- {1-[bis-(4-chlorophényl)-méthyl]-azétidin-3-yl}-amide,
(5-méthylsulfonyl-3-méthyl-4-vinyl-thiophène-2-carboxy)-{ 1-[bis-(4-chlorophényl)-méthyl]-azétidin-3-yl} -amide,
(RS)-N-{1-[(4-chloro-phényl)-pyridin-3-yl-méthyl]-azétidin-3-yl}-3,5-difluoro-benzènesulfonamide,
(RS)-N-{1-[(4-chloro-phényl)-pyrimidin-5-yl-méthyl]-azétidin-3-yl}-3,5-difluoro-benzenesulfonamide,
N-{1-[bis-(4-chlorophényl)méthyl]-azétidin-3-yl}-N-(6-chloropyrid-2-yl)-méthyl-sulfonamide,
N-{1-[bis-(4-chlorophényl)méthyl]azétidin-3-yl}-N-(6-éthylpyrid-2-yl)-méthyl-sulfonamide,
N-{1-[bis-(4-chlorophényl)méthyl]azétidin-3-yl}--N-quinol-6-yl-méthyl-sulfonamide,
N-{1-[bis-(4-chlorophényl)méthyl]azétidin-3-yl}-N-quinol-5-yl-méthyl-sulfonamide,
N-{1-[bis-(4-chlorophényl)méthyl]azétidin-3-yl}-N-isoquinol-5-yl-méthyl-sulfonamide,
N-{1-[bis-(4-chlorophényl)méthyl]azétidin-3-yl}-N-pyrid-3-yl-méthyl-sulfonamide,
N-{1-[bis-(4-chlorophényl)méthyl]azétidin-3-yl}-N-(1-oxyde-pyrid-3-yl)-méthylsulfonamide,
N-(1R,2S,4S)-bicyclo[2,2,1]hept-2-yl-N-{1-[bis-(4-chlorophényl)méthyl] azétidin-3-yl}-méthylsulfonamide,
N-(1R,2R,4S)-bicyclo[2,2,1]hept-2-yl-N-{1-[bis-(4-chlorophényl)méthyl] azétidin-3-yl}-méthylsulfonamide,
N-{1-[bis-(4-chlorophényl)méthyl]azétidin-3-yl}-N-(3,5-difluorophényl)-méthylsulfonamide,
N-{1-[bis-(4-chlorophényl)méthyl]azétidin-3-yl}-N-(thiazol-2-yl)-méthyl sulfonamide,
N-{1-[bis-(4-chlorophényl)-méthyl]-azétidin-3-yl}-N-(3-méthoxyphényl)-méthylsulfonamide,
N-{1-[bis-(4-chlorophényl)-méthyl]-azétidin-3-yl}-N-(3-hydroxyphényl)-méthylsulfonamide,
N-{1-[bis-(4-chlorophényl)-méthyl]-azétidin-3-yl}-N-(3-hydroxyméthyl-phényl)-méthylsulfonamide,
N-{1-[bis-(4-chlorophényl)-méthyl]-azétidin-3-yl}-N-(méthylsulfonyl)-3-aminobenzoate d'éthyle,
N-{1-[bis-(4-chlorophényl)méthyl]azétidin-3-yl}-N-(1-isobutyl-pipérid-4-yl)-méthylsulfonamide,
N-benzyl-N- {1-[bis(4-chlorophényl)méthyl]azétidin-3-yl} amine,
N-{1-[bis(4-chlorophényl)méthyl]azétidin-3-yl}-N-(3,5-difluorobenzyl)amine,
N-{1-[bis(4-chlorophényl)méthyl]azétidin-3-yl}-N-(3,5-difluorobenzyl)méthylsulfonamide,
N-{1-[bis(4-chlorophényl)méthyl]azétidin-3-yl}-N-(pyrid-3-yl-méthyl)-méthylsulfonamide,
N-{1-[bis-(4-fluoro-phényl)-méthyl]-azétidin-3-yl}-N-(3,5-difluorophényl)-méthylsulfonamide,
(RS)-N- {1-[(4-chlorophényl)-pyrid-3-yl-méthyl]-azétidin-3-yl}-N-(3,5-difluorophényl)-méthylsulfonamide,
(R)-N- {1-[(4-chlorophényl)-pyrid-3-yl-méthyl]-azétidin-3-yl}-N-(3,5-difluorophényl)-méthylsulfonamide,
(S)-N-{1-[(4-chlorophényl)-pyrid-3-yl-méthyl]-azétidin-3-yl}-N-(3,5-difluorophényl)-méthylsulfonamide,
(RS)-N-{1-[(4-chlorophényl)-pyrid-4-yl-méthyl]-azétidin-3-yl}-N-(3,5-difluorophényl)-méthylsulfonamide,
(R)-N- {1-[(4-chlorophényl)-pyrid-4-yl-méthyl]-azétidin-3-yl}-N-(3,5-difluorophényl)-méthylsulfonamide,
(S)-N- {1-[(4-chlorophényl)-pyrid-4-yl-méthyl]-azétidin-3-yl}-N-(3,5-difluorophényl)-méthylsulfonamide,
(RS)-N-{1-[(4-chlorophényl)-pyrimidin-5-yl-méthyl]-azétidin-3-yl}-N-(3,5-difluorophényl)-méthylsulfonamide,
(R)-N- {1-[(4-chlorophényl)-pyrimidin-5-yl-méthyl]-azétidin-3-yl}-N-(3,5-difluorophényl)-méthylsulfonamide,
(S)-N- {1-[(4-chlorophényl)-pyrimidin-5-yl-méthyl]-azétidin-3-yl}-N-(3,5-difluorophényl)-méthylsulfonamide,
N- {1-[bis-(4-chlorophényl)méthyl]azétidin-3-yl}-N-(3,5-difluorophényl)-benzylsulfonamide,
leurs isomères optiques et leurs sels pharmaceutiquement acceptables.

Comme exemples de sels pharmaceutiquement acceptables des dérivés d'azétidine, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-oxynaphtoate, nitrate, oxalate, pamoate, phosphate, salicylate, succinate, sulfate, tartrate, théophyllinacétate et p-toluènesulfonate.

Les dérivés d'azetidine sont synthétisés selon les méthodes générales suivantes :

Les composés de formule (I) pour lesquels R représente un radical CR₁R₂ dans lequel R₁ représente un atome d'hydrogène et R₂ représente un radical C(R₈)(R₁₁)(R₁₂) dans lequel R₈ représente un atome d'hydrogène, R₁₁ représente un radical -SO₂-Ar₁, -SO₂-Het₁ ou -SO₂alk et R₁₂ représente un atome d'hydrogène ou un radical Ar₁ ou Het₁ et les composés de formule (I) pour lesquels R représente un radical C=C(R₅)SO₂R₆ ou C=C(R₇)SO₂alk peuvent être préparés selon le schéma réactionnel suivant : 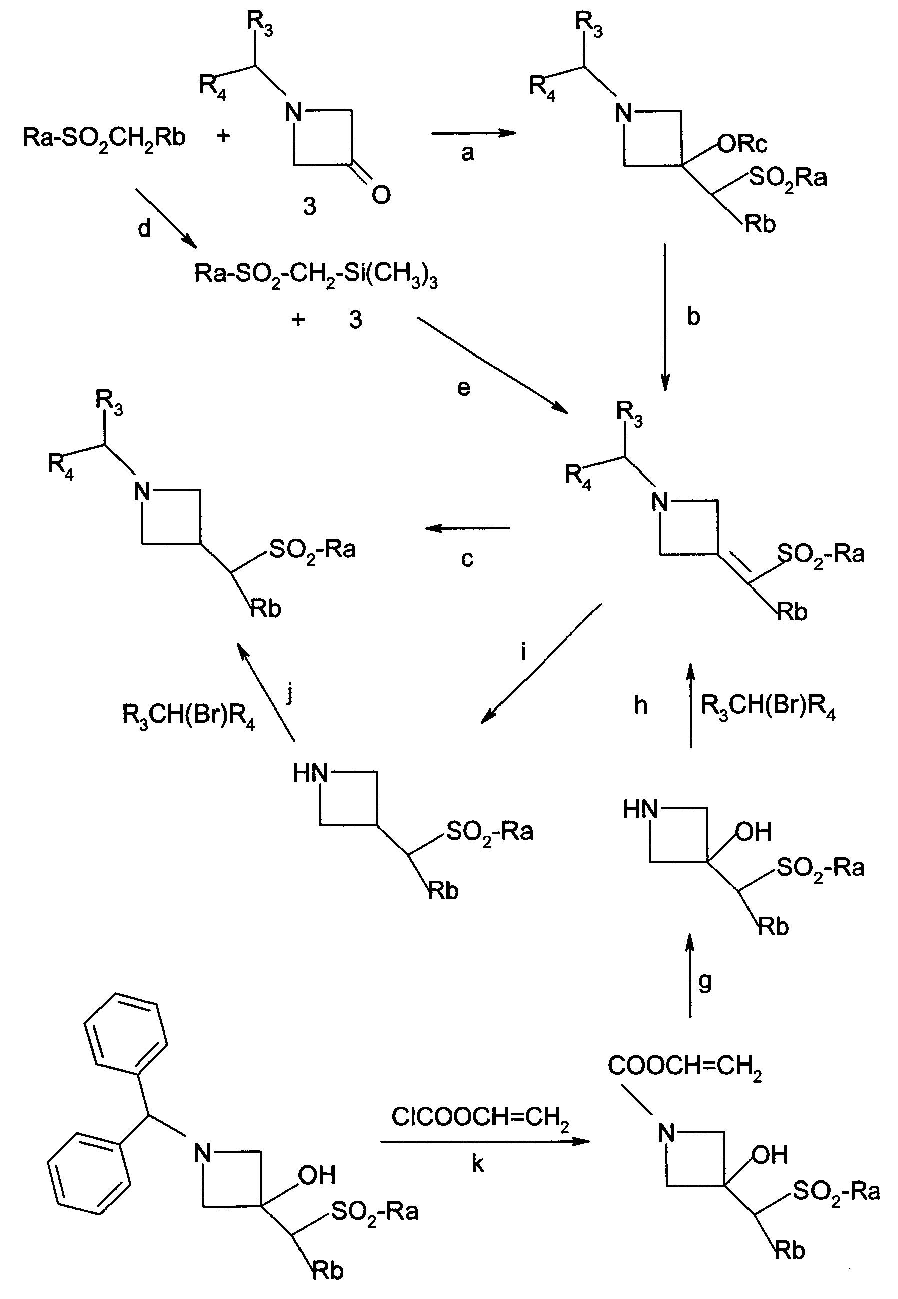 dans ces formules, soit Ra représente un radical alkyle, Het₁ ou Ar₁ et Rb représente un atome d'hydrogène ou un radical Ar₁ ou Het₁, soit Ra représente un radical Ar₁ ou Het₁ et Rb représente un atome d'hydrogène ou un radical alkyle, soit Ra représente un radical alkyle et Rb représente un radical cycloalkyle, hétérocycloalkyle ou hétérocyclényle éventuellement substitué par un radical -CSO-phényle, Rc représente un atome d'hydrogène ou un radical acétyle, R₃, R₄, Ar₁ et Het₁ ont les mêmes significations que dans la formule (I).

Les réactions d et e ne peuvent être utilisées que lorsque Rb est un atome d'hydrogène.

La réaction a s'effectue généralement au sein d'un solvant inerte tel qu'un éther (tétrahydrofuranne par exemple), en présence d'une base forte telle que le tert-butyllithium, le n-butyllithium, le diisopropylamidure de lithium, le tert-butylate de potassium, à une température comprise entre -70°C et -15°C.

La réaction de déshydratation b s'effectue généralement par toute méthode de déshydratation connue de l'homme de l'art permettant de déshydrater un alcool pour obtenir l'alcène correspondant. De préférence, on prépare le dérivé acétyloxy par action de chlorure d'acétyle, au sein d'un solvant inerte tel que la pyridine, le tétrahydrofuranne, le dioxanne, un solvant chloré (dichlorométhane, chloroforme par exemple), à une température comprise entre 5°C et 20°C puis on traite avec une base telle qu'un hydroxyde de métal alcalin (soude par exemple), un carbonate de métal alcalin (carbonate de sodium ou de potassium par exemple), une amine telle qu'une trialkylamine (triéthylamine par exemple), la 4-diméthylaminopyridine, le diaza-1,8-bicyclo[5.4.0]undécène-7, à une température comprise entre 0°C et la température d'ébullition du milieu réactionnel. L'acétyloxy intermédiaire peut être isolé ou non. L'acétyloxy peut aussi être préparé directement dans le milieu réactionnel de la réaction a.

La réduction c s'effectue généralement au sein d'un solvant inerte tel qu'un alccol aliphatique (1-4C) (méthanol par exemple), un solvant chloré (chloroforme, dichlorométhane par exemple) ou un mélange de ces solvants, en présence de NaBH₄, à une température comprise entre 0°C et la température d'ébullition du milieu réactionnel

La réaction d s'effectue par action de chlorure de triméthylsilyle, au sein d'un solvant inerte tel qu'un éther (tétrahydrofuranne par exemple), en présence de n-butyllithium, à une température de -70°C.

La réaction e s'effectue généralement au sein d'un solvant inerte tel qu'un éther (tétrahydrofuranne par exemple), en présence d'une base forte telle que le tert-butyllithium, le n-butyllithium, le diisopropylamidure de lithium, le tert-butylate de potassium, à une température comprise entre -70°C et -15°C.

L'hydrolyse g s'effectue au sein d'un solvant inerte tel qu'un éther (dioxane par exemple), au moyen d'acide chlorhydrique, à une température voisine de 20°C.

Les réactions h et j s'effectuent de préférence au sein d'un solvant inerte tel que l'acétonitrile, en présence d'une base tel qu'un carbonate de métal alcalin (carbonate de potassium par exemple), à la température d'ébullition du milieu réactionnel.

La réaction i s'effectue sous atmosphère d'hydrogène, en présence d'un catalyseur tel que le palladium ou l'un de ses dérivés, au sein d'un solvant inerte tel que le méthanol ou l'éthanol, à une température comprise entre 15°C et 60°C.

La réaction k s'effectue au sein d'un solvant inerte tel qu'un solvant chloré (dichlorométhane, chloroforme par exemple), à une température comprise entre 0°C et la température d'ébullition du mélange réactionnel.

Les dérivés R₃CH(Br)R₄ sont commercialisés ou peuvent être obtenus par application ou adaptation de la méthode décrite par BACHMANN W.E., J. Am. Chem. Soc., 2135 (1933). Généralement, on brome l'alcool correspondant R₃CHOHR₄ au moyen d'acide bromhydrique, au sein de l'acide acétique, à une température comprise entre 0°C et la température d'ébullition du milieu réactionnel.

Les alcools correspondants R₃CHOHR₄ sont commercialisés ou peuvent être obtenus par application ou adaptation des méthodes décrites par PLASZ A.C. et coll., J. Chem. Soc. Chem. Comm., 527 (1972).

Les intermédiaires suivants peuvent être obtenus par application ou adaptation des méthodes décrites dans les exemples. Notamment, on opère selon les schémas réactionnels suivants : 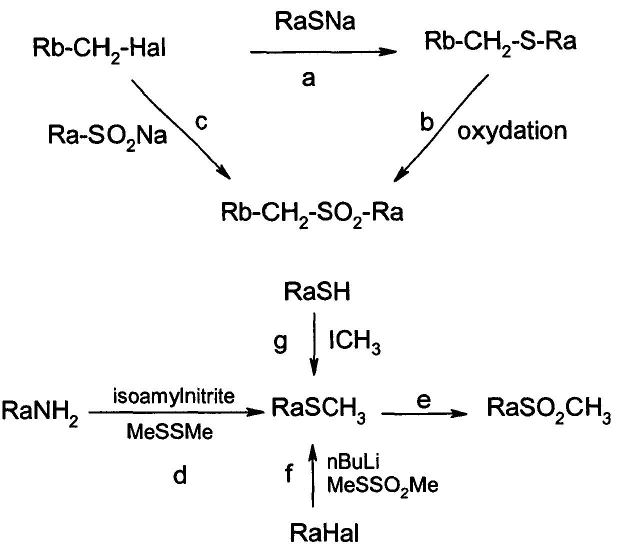 dans ces formules Hal représente un atome d'halogène et, de préférence, chlore, brome ou iode.

La réaction a s'effectue généralement au sein d'un solvant inerte tel que le diméthylformamide ou un alcool aliphatique 1-4C, à une température comprise entre 20°C et 30°C.

Les réactions b et e s'effectuent par toutes méthodes connues permettant d'oxyder un dérivé soufré sans toucher au reste de la molécule comme celles décrites par M. HUDLICKY, Oxidations in Organic Chemistry, ACS Monograph, 186, 252-263 (1990). Par exemple, on opère par action d'un peroxyacide organique ou un sel d'un tel peroxyacide (acides peroxycarboxyliques ou peroxysulfoniques, notamment l'acide peroxybenzoïque, l'acide 3-chloroperoxybenzoïque, l'acide 4-nitroperoxybenzoïque, l'acide peroxyacétique, l'acide trifluoroperoxyacétique, l'acide peroxyformique, l'acide monoperoxyphtalique) ou les peracides minéraux ou un sel d'un tel acide (par exemple l'acide periodique ou persulfurique), au sein d'un solvant inerte tel qu'un solvant chloré (chloroforme, dichlorométhane par exemple), à une température comprise entre 0 et 25°C. On peut utiliser également le peroxyde d'hydrogène, éventuellement en présence d'un oxyde métallique (tungstate de sodium) ou un periodate (periodate de sodium par exemple), au sein d'un solvant inerte tel qu'un alcool aliphatique 1-4C (méthanol, éthanol par exemple), l'acide acétique, l'eau ou un mélange de ces solvants, à une température comprise entre 0 et 60°C. Il est également possible d'opérer au moyen de tertiobutylhydroperoxyde en présence de tétraisopropylate de titane au sein d'un alcool aliphatique 1-4C (méthanol, éthanol par exemple) ou un mélange eau-alcool, à une température voisine de 25°C ou au moyen d'oxone^{R} (peroxymonosulfate de potassium), au sein d'un alcool aliphatique 1-4C (méthanol, éthanol par exemple), en présence d'eau, d'acide acétique ou d'acide sulfurique, à une température voisine de 20°C.

La réaction c s'effectue de préférence au sein d'un solvant inerte tel qu'un alcool aliphatique 1-4C (méthanol, éthanol par exemple), à une température comprise entre 20°C et la température d'ébullition du milieu réactionnel.

La réaction d s'effectue sous atmosphère inerte (argon), à une température comprise entre 50°C et la température d'ébullition du milieu réactionnel.

La réaction f s'effectue généralement au sein d'un solvant inerte tel que le tétrahydrofuranne ou un éther aliphatique (éther éthylique par exemple), à une température voisine de -70°C.

La réaction g s'effectue généralement au sein d'un solvant inerte tel que le diméthylformamide, un éther aliphatique (éther éthylique par exemple) ou un alcool aliphatique 1-4C, en présence d'une base (hydrure de sodium par exemple), à une température comprise entre 0°C et 60°.

Les dérivés de formule Rb-CH₂-Hal sont commercialisés ou peuvent être obtenus par application ou adaptation des méthodes décrites dans les exemples. En particulier, on halogène le dérivé méthylé ou l'alcool correspondant, au moyen d'un agent d'halogénation tel que l'acide bromhydrique, au sein de l'acide acétique, à une température voisine de 20°C ou le N-bromo ou N-chlorosuccinimide en présence de peroxyde de benzoyle, au sein d'un solvant inerte tel que le tétrachlorométhane, à la température d'ébullition du milieu réactionnel. Les dérivés méthylés ou les alcools correspondants sont commercialisés ou peuvent être obtenus selon les méthodes décrites par BRINE G. A. et coll., J. Heterocyl. Chem, 26, 677 (1989) et NAGARATHNAM D., Synthesis, 8, 743 (1992) et dans les exemples.

Les azétidinones de formule 3 peuvent être obtenues par application ou adaptation des méthodes décrites par KATRITZKY A.R et coll., J. Heterocycl. Chem., 271 (1994), ou DAVE P.R., J. Org. Chem., 61, 5453 (1996) et dans les exemples. On opère généralement selon le schéma réactionnel suivant : 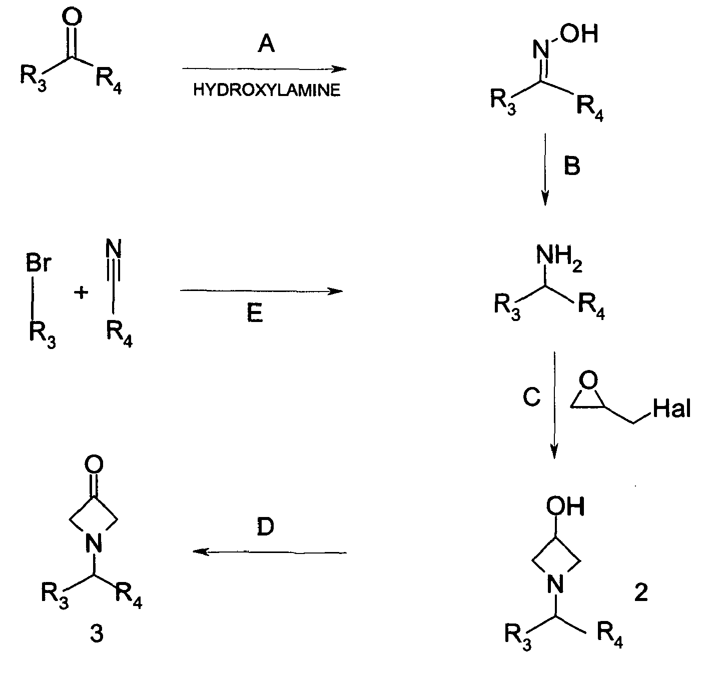 dans ces formules R₃ et R₄ ont les mêmes significations que dans la formula (I) et Hal représente un atome de chlore ou de brome.

Dans l'étape A, on opère de préférence au sein d'un solvant inerte tel qu'un alcoll aliphatique 1-4C (éthanol, méthanol par exemple), éventuellement en présence d'un hydroxyde de métal alcalin, à la température d'ébullition du milieu réactionnel.

Dans l'étape B, la réduction s'effectue généralement, au moyen d'hydrure de lithium et d'aluminium, au sein du tétrahydrofuranne à la température d'ébullition du milieu réactionnel.

Dans l'étape C, on opère de préférence au sein d'un solvant inerte tel qu'un alcool aliphatique 1-4C (éthanol, méthanol par exemple), en présence d'hydrogénocarbonate de sodium, à une température comprise entre 20°C et la temperature d'ébullition du milieu réactionnel.

Dans l'étape D on oxyde de préférence au sein de DMSO, au moyen du complexe trioxyde de soufre-pyridine, à une température voisine de 20°C ou au moyen de di-méthylsulfoxyde, en présence de chlorure, d'oxalyle et de triéthylamine, à une température comprise entre -70 et -50°C.

Dans l'étape E, on opère selon la méthode décrite par GRISAR M. et colls. dans J. Med. Chem., 885 (1973). On forme le magnésien du dérivé bromé puis on fait réagir le nitrile, au sein d'un éther tel que l'éther éthylique, à une température comprise entre 0°C et la température d'ébullition du milieu réactionnel. Après hydrolyse avec un alcool, l'imine intermédiaire est réduite *in situ* par du borohydrure de sodium à une température comprise entre 0°C et la température d'ébullition du milieu réactionnel.

Les dérivés R₃-CO-R₄ sont commercialisés ou peuvent être obtenus par application ou adaptation des méthodes décrites par KUNDER N.G. et coll. J. Chem. Soc. Perkin Trans 1, 2815 (1997); MORENO-MARRAS M., Eur. J. Med., 23 (5) 477 (1988); SKINNER et coll., J. Med. Chem., 14 (6) 546 (1971); HURN N.K., Tet. Lett., 36 (52) 9453 (1995); MEDICA A. et coll., Tet Lett., 24 (28) 2901 (1983); RIECKE R.D. et coll., J. Org. Chem., 62 (20) 6921 (1997); KNABE J. et coll., Arch. Pharm., 306 (9) 648 (1973); CONSONNI R. et coll., J. Chem. Soc. Perkin Trans 1, 1809 (1996); FR-96-2481 et JP-261393.

Les dérivés R₃Br sont commercialisés ou peuvent être obtenus par application ou adaptation des méthodes décrites par BRANDSMA L. et coll., Synth. Comm., 20 (11) 1967 et 3153 (1990); LEMAIRE M. et coll., Synth. Comm., 24 (1) 95 (1994); GODA H. et coll., Synthesis, 9 849 (1992); BAEUERLE P. et coll., J. Chem. SOC. Perkin Trans 2, 489 (1993).

Les dérivés R₄CN sont commercialisés ou peuvent être obtenus par application ou adaptation des méthodes décrites par BOUYSSOU P. et coo., J. Het. Chem., 29 (4) 895 (1992); SUZUKI N. et coll., J. Chem. Soc. Chem. Comm., 1523 (1984); MARBURG S. et coll., J. Het. Chem., 17 1333 (1980); PERCEC V. et coll., J. Org. Chem., 60 (21) 6895 (1995).

Les composés de formule (I) pour lesquels R représente un radical CR₁R₂ dans lequel R₁ représente un atome d'hydrogène et R₂ représente un radical C(R₈)(R₉)(R₁₀) dans lequel R₈ représente un atome d'hydrogène, R₉ représente un radical -CO-NR₂₆R₂₇, -COOH, -COOalk, -CH₂OH, -NHCOOalk ou -NH-CO-NH-alk et R₁₀ représente un radical Ar₁ et Het₁ peuvent être préparés selon le schéma réactionnel suivant : 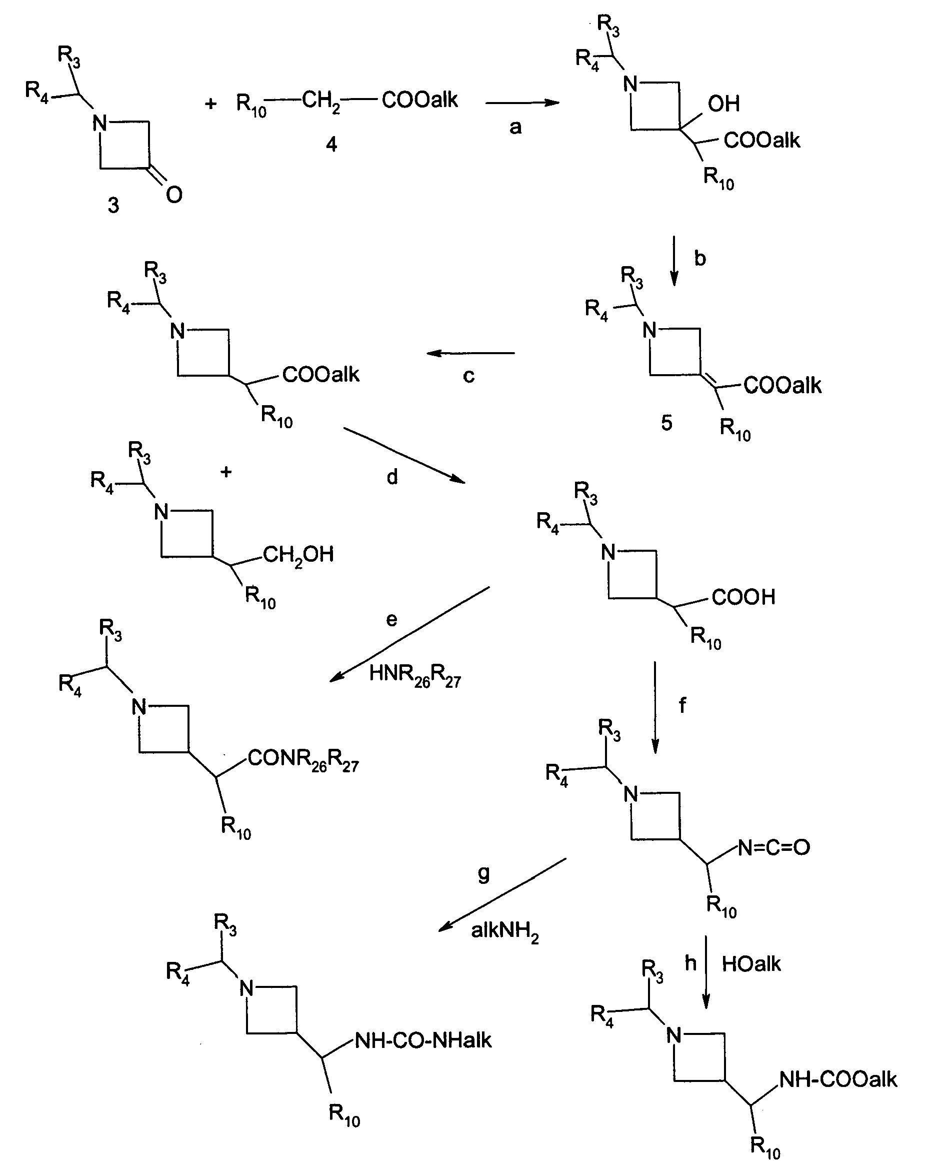 dans ces formules R₃, R₄, R₁₀, R₂₆ et R₂₇ ont les mêmes significations que dans la formule (I) et alk représente un radical alkyle.

Les dérivés de formule 4 sont commercialisés ou peuvent être obtenus par estérification des acides correspondants éventuellement sous une forme activée telle que le chlorure d'acide. Les acides sont commercialisés ou peuvent être obtenus à partir des dérivés méthylés correspondants selon la méthode décrite par JP. HANSEN et coll., J. Heteocycl., 10, 711 (1973).

La réaction a s'effectue généralement au sein d'un solvant inerte tel qu'un éther (tétrahydrofuranne par exemple), en présence d'une base forte telle que le tert-butyllithium, le n-butyllithium, le diisopropylamidure de lithium, le tert-butylate de potassium, à une température comprise entre -70°C et -15°C.

La réaction b s'effectue généralement par toute méthode de déshydratation connue de l'homme de l'art permettant de déshydrater un alcool pour obtenir l'alcène correspondant et notamment les méthodes décrites précédemment.

La réduction c s'effectue généralement au sein d'un solvant inerte tel qu'un alcool aliphatique (1-4C) tel que le méthanol, un solvant chloré tel que le chloroforme, le dichlorométhane ou un mélange de ces solvants, en présence de NaBH₄, à une température comprise entre 0°C et la température d'ébullition du milieu réactionnel.

La réaction d s'effectue par toute méthode connue de l'homme de l'art permettant de passer d'un ester à l'acide correspondant sans toucher au reste de la molécule. On opère de préférence, au sein d'un solvant inerte tel que le dioxanne, en présence d'acide chlorhydrique, à la température d'ébullition du milieu réactionnel.

La réaction e s'effectue par toute méthode connue de l'homme de l'art permettant de passer d'un acide ou un dérivé réactif de cet acide à un carboxamide sans toucher au reste de la molécule. De préférence, lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel. Lorsque l'on met en oeuvre un dérivé réactif de l'acide, il est possible de faire réagir l'anhydride, un anhydride mixte ou un ester (qui peut être choisi parmi les esters activés ou non de l'acide); on opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

La réaction f s'effectue par réarrangement de CURTIUS, en présence de diphénylphosphorazide et de triéthylamine, au sein du toluène, à une température voisine de 50°C.

Pour les réactions g et h, on opère directement au sein du milieu réactionnel de l'étape g à une température voisine de 20°C.

Les composés de formule (I) pour lesquels R représente un radical CR₁R₂ dans lequel R₁ est un atome d'hydrogène et R₂ représente un radical -C(R₈)(R₉)(R₁₀) pour lequel R₈ est un atome d'hydrogène, R₉ est un radical -CH₂-NHR₂₈ et R₁₀ représente un radical Ar₁ ou Het₁, peuvent être préparés selon le schéma réactionnel suivant :

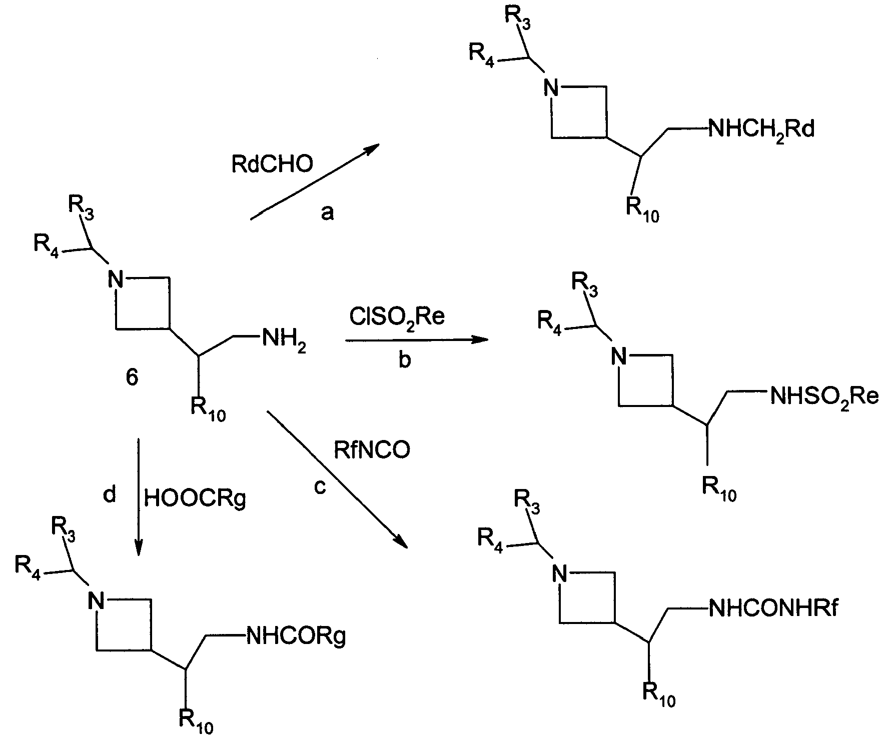

Dans ces formules R₃, R₄ et R₁₀ ont les mêmes significations que dans la formule (I), Rd représente un radical alkyle ou phényle, Re représente un radical alkyle, Rf représente un radical alkyle, Rg représente un radical alkyle, cycloalkylalkyle, cycloalkyle, -(CH₂)ₙOH, n est égal à 1, 2, 3.

L'étape a s'effectue généralement au sein d'un solvant inerte tel qu'un alcool aliphatique (1-4C) (méthanol par exemple), un solvant chloré (dichlorométhane, dichloroéthane par exemple) ou le tétrahydrofuranne, en présence d'une base telle que NaBH(OCOCH₃)₃, à une température voisine de 20°C.

L'étape b s'effectue généralement au sein d'un solvant inerte tel qu'un solvant halogéné (dichlorométhane par exemple), en présence d'une base organique telle que la triéthylamine, la diméthylaminopyridine, à une température comprise entre 0°C et la température d'ébullition du milieu réactionnel.

L'étape c s'effectue généralement au sein d'un solvant inerte tel que le tétrahydrofuranne, le diméthylformamide, un solvant chloré (chloroforme, dichloro-1,2 éthane par exemple), un solvant aromatique (benzène, toluène par exemple), à une température comprise entre 10°C et la température d'ébullition du milieu réactionnel.

L'étape d s'effectue par toute méthode connue de l'homme de l'art permettant de passer d'un acide ou un dérivé réactif de cet acide à un carboxamide sans toucher au reste de la molécule et notamment les méthodes préférées décrites précédemment.

Les dérivés 6 peuvent être obtenus selon le schéma réactionnel suivant :

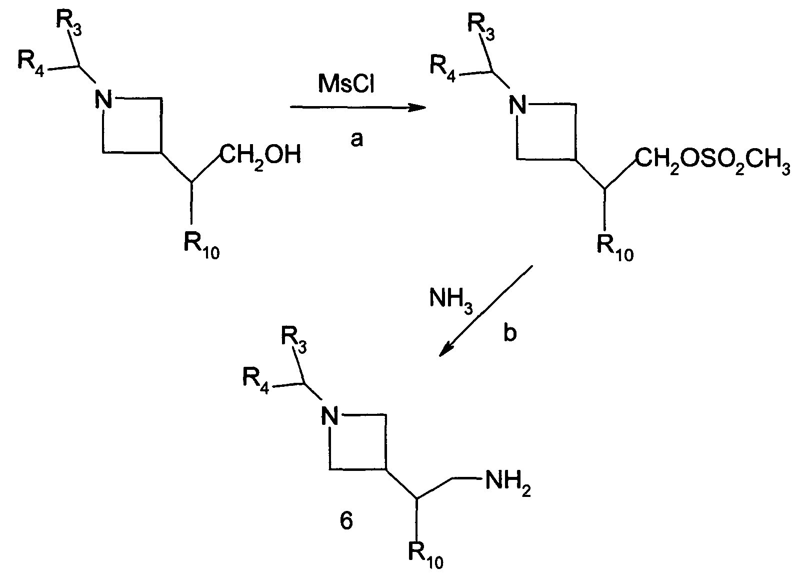

Dans ces formules R₃, R₄ et R₁₀ ont les mêmes significations que dans la formule (I) et Ms est un radical méthylsulfonyloxy.

L'étape a s'effectue généralement au sein d'un solvant inerte tel que le tétrahydrofuranne, en présence de triéthylamine, à une température comprise entre 10 et 20°C.

L'étape b s'effectue généralement avec de l'ammoniaque liquide dans le méthanol, en autoclave, à une température voisine de 60°C.

Les composés de formule (I) dans laquelle R représente un radical CR₁R₂ dans lequel R₁ est un atome d'hydrogène et R₂ est un radical -CONR₁₃R₁₄ peuvent être préparés selon le schéma réactionnel suivant :

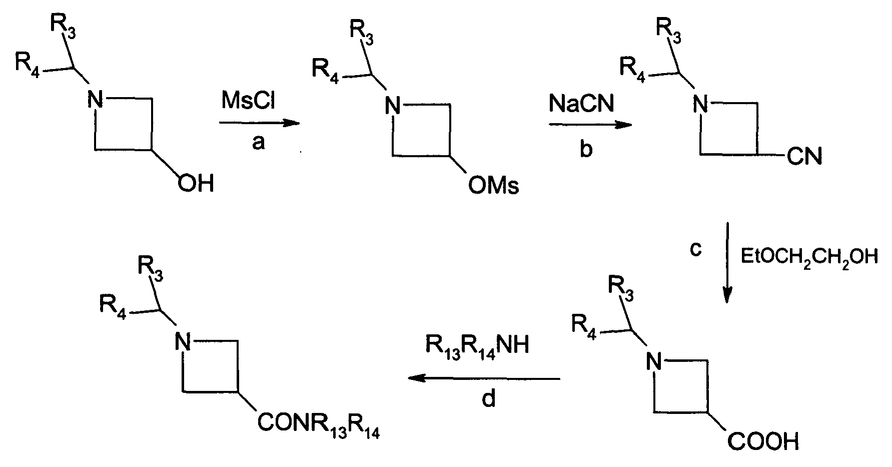

Dans ces formules R₃, R₄, R₁₃ et R₁₄ ont les mêmes significations que dans la formule (I), Ms représente un radical méthylsulfonyloxy et Et représente éthyle.

L'étape a s'effectue en présence de triéthylamine, au sein d'un solvant inerte tel qu'un éther (tétrahydrofuranne par exemple), à une température voisine de 0°C.

L'étape b s'effectue généralement au sein d'un solvant inerte tel qu'un mélange d'eau et de diméthylformamide, à une température comprise entre 30 et 75°C.

L'étape c s'effectue par toute méthode connue de l'homme de l'art permettant de passer d'un cyané à l'acide correspondant sans toucher au reste de la molécule. De préférence, on opère au moyen de potasse au sein d'un alcool aliphatique (1-4C) (éthanol par exemple) ou en milieu aqueux, à la température d'ébullition du milieu réactionnel.

L'étape d s'effectue par toute méthode connue de l'homme de l'art permettant de passer d'un acide ou un dérivé réactif de cet acide à un carboxamide sans toucher au reste de la molécule molécule et notamment les méthodes préférées décrites précédemment.

Les composés de formule (I) pour lesquels R représente un radical CR₁R₂ dans lequel R₁ est un atome d'hydrogène et R₂ est un radical -CH₂-CONR₁₃R₁₄ peuvent être préparés selon le schéma réactionnel suivant :

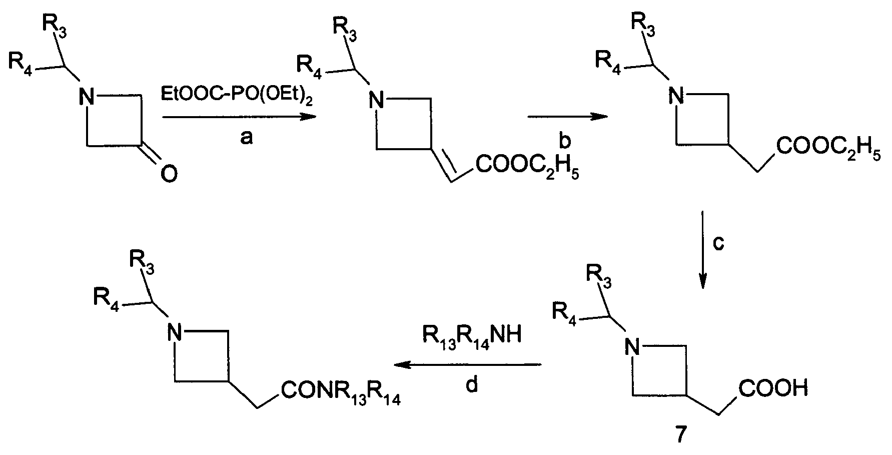

Dans ces formules R₃, R₄, R₁₃ et R₁₄ ont les mêmes significations que dans la formule (I) et Et représente un radical éthyle.

La réaction a s'effectue généralement au sein d'un solvant inerte tel que le tétrahydrofuranne, en présence d'une base telle que l'hydrure de sodium, ou un carbonate de métal alcalin (carbonate de potassium par exemple), à une température comprise entre 20°C et la température d'ébullition du milieu réactionnel.

La réaction b s'effectue généralement au moyen de NaBH₄, au sein de l'éthanol, à une température voisine de 0°C.

La réaction c s'effectue par toute méthode connue de l'homme de l'art permettant de passer d'un ester à l'acide correspondant sans toucher au reste de la molécule. On opère de préférence, au sein d'un solvant inerte tel que le dioxanne, en présence d'acide chlorhydrique, à la température d'ébullition du milieu réactionnel.

La réaction d s'effectue par toute méthode connue de l'homme de l'art permettant de passer d'un acide ou un dérivé réactif de cet acide à un carboxamide sans toucher au reste de la molécule et notamment les méthodes préférées décrites précédemment.

Les intermédiaires 7 peuvent également être obtenus par synthèse malonique selon le schéma réactionnel suivant :

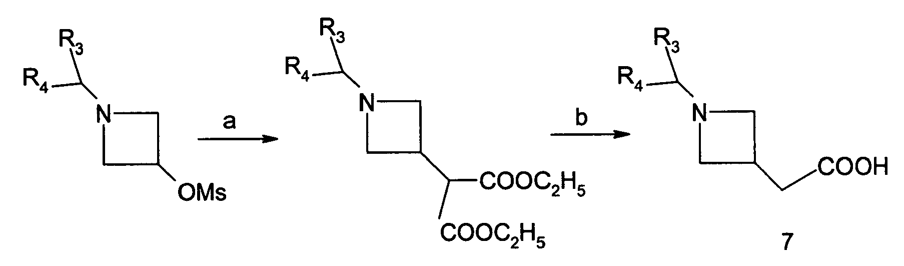

Dans ces formules Ms représente un radical méthylsulfonyloxy, R₃ et R₄ ont les mêmes significations que dans la formule (I).

La réaction a s'effectue généralement par action de diéthylmalonate, au sein d'un solvant inerte tel que le tétrahydrofuranne, en présence d'éthylate de sodium fraîchement préparé, à la température d'ébullition du milieu réactionnel.

La réaction b s'effectue généralement en solution aqueuse d'acide chlorhyrique à la température d'ébullition du milieu réactionnel.

Les composés 8 peuvent également être obtenus selon le schéma réactionnel suivant :

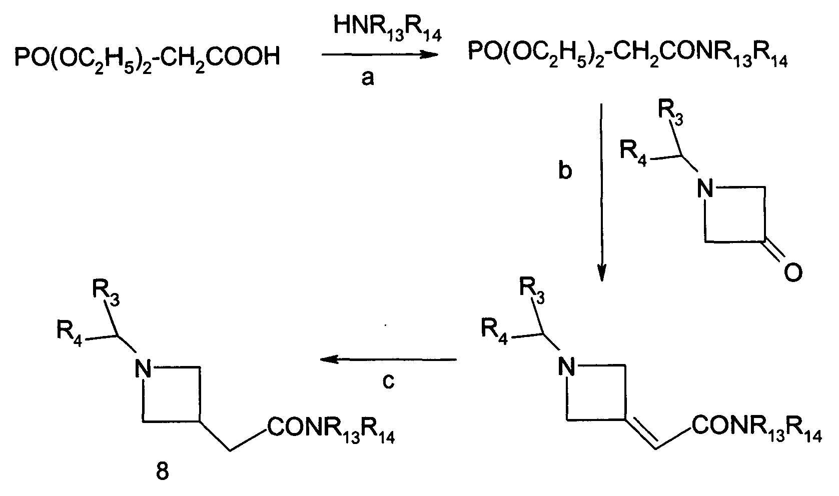

Dans ces formules R₃, R₄, R₁₃ et R₁₄ ont les mêmes significations que dans la formule (I).

L'étape a s'effectue par toute méthode connue de l'homme de l'art permettant de passer d'un acide ou un dérivé réactif de cet acide à un carboxamide sans toucher au reste de la molécule molécule et notamment les méthodes préférées décrites précédemment.

L'étape b s'effectue généralement au sein d'un solvant inerte tel que le tétrahydrofuranne, en présence d'une base telle que l'hydrure de sodium ou le carbonate de potassium à une température comprise entre 20°C et la température d'ébullition du mélange réactionnel.

La réduction de l'étape c s'effectue généralement au moyen de NaBH₄, au sein de l'éthanol, à une tempéraure de voisine de 20°C.

Les composés de formule (I) pour lesquels R représente un radical CR₁R₂ dans lequel R₁ est un atome d'hydrogène et R₂ représente un radical -SOR₆ ou -SO₂R₆ peuvent être préparés selon le schéma réactionnel suivant :

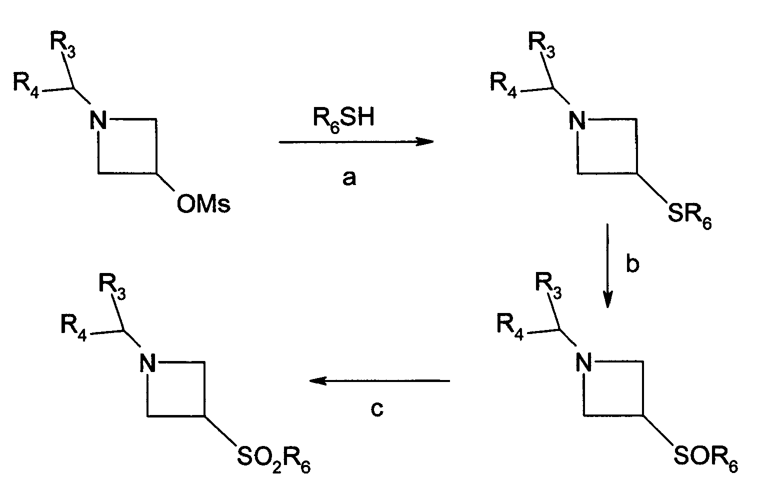

Dans ces formules R₃, R₄ et R₆ ont les mêmes significations que dans la formule (I) et Ms est un radical méthylsulfonyloxy.

L'étape a s'effectue généralement dans un solvant inerte tel que le tétrahydrofuranne en présence d'une base minérale tel que l'hydrure de sodium, à une température comprise entre 0°C et la température d'ébullition du milieu réactionnel.

L'étape b s'effectue généralement par toute méthode de l'homme de l'art d'oxydation d'un dérivé soufré comme celles décrites par M. HUDLICKY, Oxidations in Organic Chemistry, ACS Monograph, 186, 252-263 (1990). Par exemple, on opère par action d'un peroxyacide organique ou un sel d'un tel peroxyacide (acides peroxycarboxyliques ou peroxysulfoniques, notamment l'acide peroxybenzoïque, l'acide 3-chloroperoxybenzoïque, l'acide 4-nitroperoxybenzoïque, l'acide peroxyacétique, l'acide trifluoroperoxyacétique, l'acide peroxyformique, l'acide monoperoxyphtalique) ou les peracides minéraux ou un sel d'un tel acide (par exemple l'acide periodique ou persulfurique), au sein d'un solvant inerte tel qu'un solvant chloré (chloroforme, dichlorométhane par exemple), à une température comprise entre 0 et 25°C ou bien au moyen d'oxone au sein d'un mélange eau-alcool (méthanol, éthanol).

L'étape c s'effectue généralement par toute méthode de l'homme de l'art d'oxydation d'un dérivé sulfinyle. De préférence, on opère par action d'un peroxyacide organique ou un sel d'un tel peroxyacide (acides peroxycarboxyliques ou peroxysulfoniques, notamment l'acide peroxybenzoïque, l'acide 3-chloroperoxybenzoïque, l'acide 4-nitroperoxybenzoïque, l'acide peroxyacétique, l'acide trifluoroperoxyacétique, l'acide peroxyformique, l'acide monoperoxyphtalique) ou bien au moyen d'oxone au sein d'un mélange eau-alcool (méthanol, éthanol).

Les composés de formule (I) pour lesquels R représente un radical CR₁R₂ dans lequel R₁ est un atome d'hydrogène et R₂ représente un radical -COR₆, ou -CO-cycloalkyle, peuvent être préparés selon le schéma réactionnel suivant :

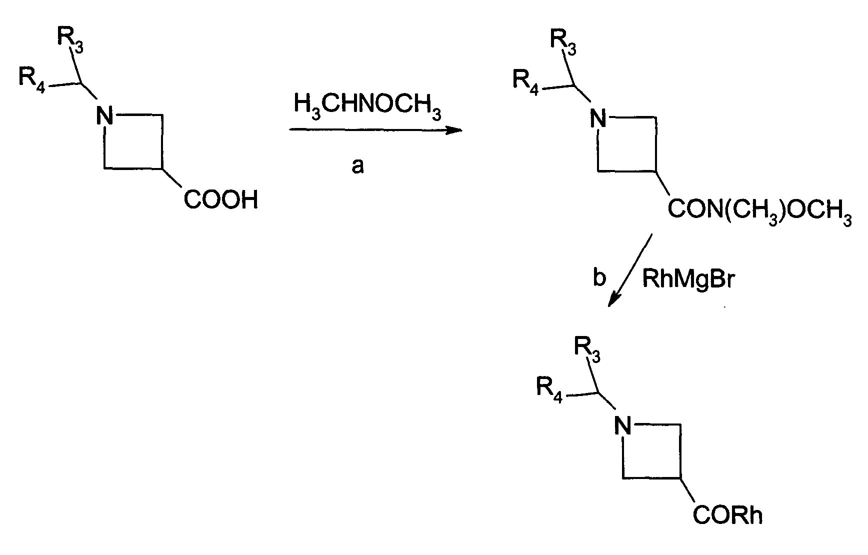

Dans ces formules, R₃ et R₄ ont les mêmes significations que dans la formule (I) et Rh a les mêmes significations que R₆ ou représente un radical cycloalkyle (3 à 10 atomes de carbone).

L'étape a s'effectue par toute méthode connue de l'homme de l'art permettant de passer d'un acide ou un dérivé réactif de cet acide à un carboxamide sans toucher au reste de la molécule molécule et notamment les méthodes préférées décrites précédemment.

L' étape b s'effectue généralement au sein d'un solvant inerte tel qu'un éther comme le tétrahydrofuranne, à une température voisine de 0°C. Les organomagnésiens sont préparés selon les méthodes connues de l'homme de l'art telles que celles décrites dans les exemples.

Les composés de formule (I) pour lesquels R₁ est un atome d'hydrogène et R₂ est un radical -C(OH)(R₆)(R₁₂), -C(OH)(R₆)(alkyle), -C(=NO-CH₂-CH=CH₂)R₆ ou - C(=NOalk)R₆ peuvent être préparés selon le schéma réactionnel suivant :

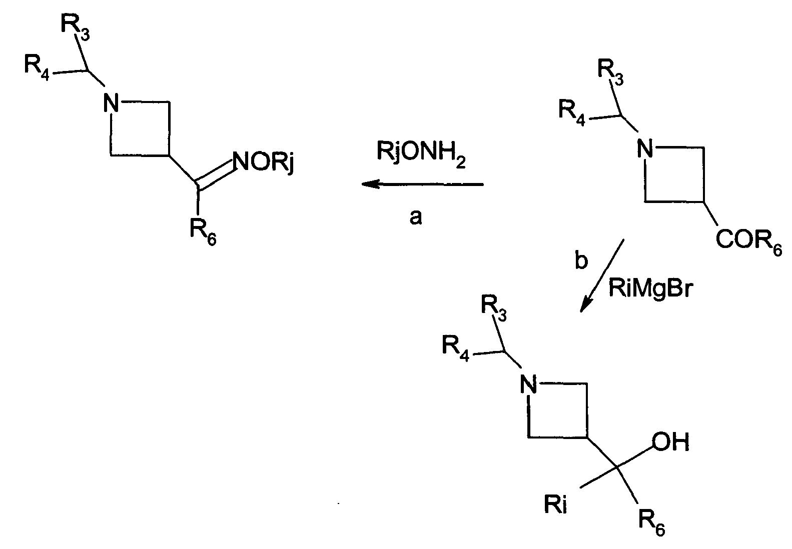

Dans ces formules, R₃, R₄ et R₆ ont les mêmes significations que dans la formule (I), Ri a les mêmes significations que R₁₂ ou représente un radical alkyle (1 à 6 atomes de carbone en chaîne droite ou ramifiée) et Rj représente un radical alkyle (1 à 6 atomes de carbone en chaîne droite ou ramifiée) ou -CH₂-CH=CH₂.

L'étape a s'effectue généralement au sein d'un solvant inerte tel qu'un alcool aliphatique (éthanol par exemple), en présence d'acétate de sodium, à une température comprise entre 20°C et la température d'ébullition du milieu réactionnel.

L' étape b s'effectue généralement au sein d'un solvant inerte tel qu'un éther comme le tétrahydrofuranne, à une température voisine de 0°C. Les organomagnésiens sont préparés selon les méthodes connues de l'homme de l'art telles que celles décrites dans les exemples.

Les composés de formule (I) pour lesquels R représente un radical CR₁R₂ dans lequel R₁ est un atome d'hydrogène et R₂ représente un radical -CH(R₆)NR₃₁R₃₂, dans lequel R₃₁ et R₃₂ sont des atomes d'hydrogène, -CH(R₆)NHSO₂alk, -CH(R₆)NHCONHalk ou -CH(R₆)NHCOR₃₁, peuvent être préparés selon le schéma réactionnel suivant :

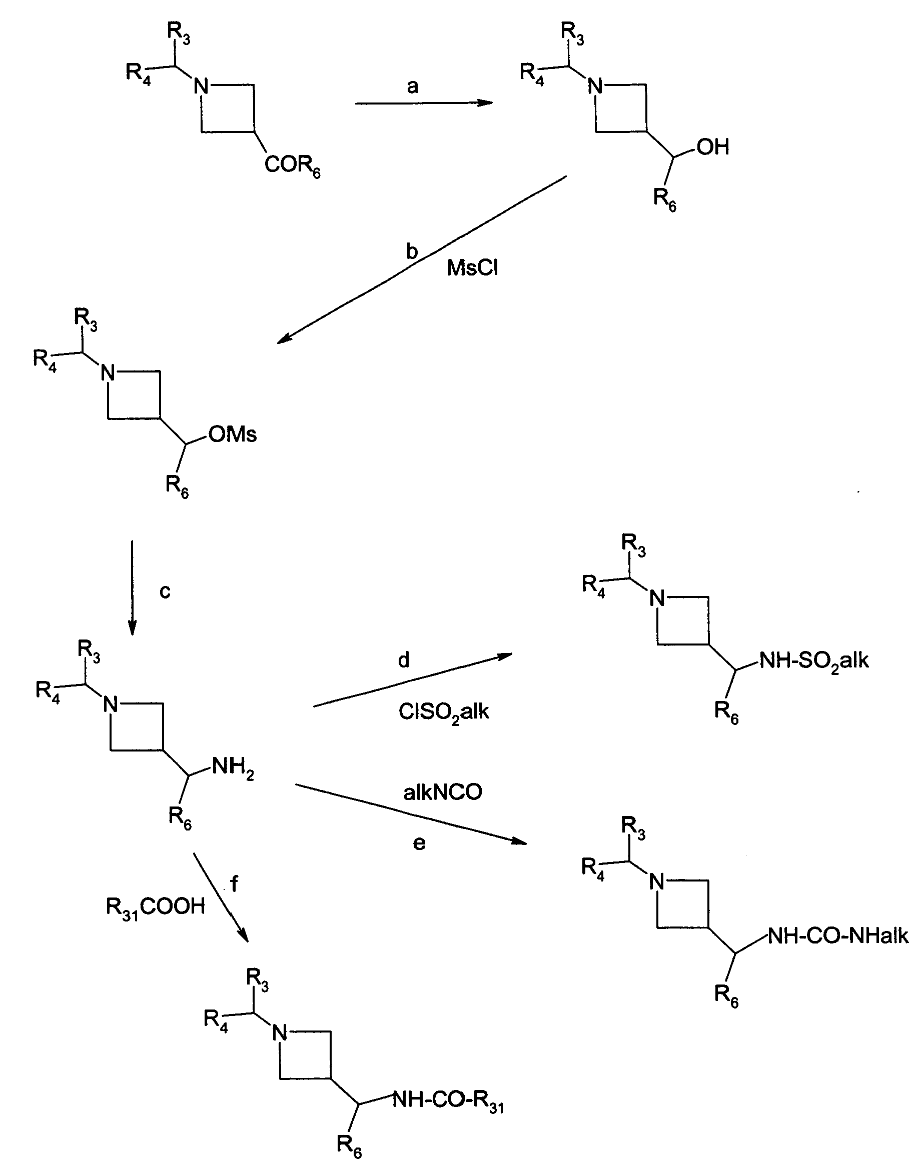

Dans ces formules R₃, R₄, R₆ et R₃₁ ont les mêmes significations que dans la formule (I), Ms représente un radical méthylsulfonyloxy, alk représente un radical alkyle.

La réaction a s'effectue généralement au moyen de NaBH₄, au sein de l'éthanol, à une tempéraure de voisine de 20°C.

L'étape b s'effectue en présence de triéthylamine, au sein d'un solvant inerte tel qu'un éther (tétrahydrofuranne par exemple), à une température voisine de 0°C.

L'étape c s'effectue au moyen d'ammoniaque liquide dans le méthanol, en autoclave à une température voisine de 60°.

L'étape d s'effectue généralement au sein d'un solvant inerte tel qu'un solvant halogéné (dichlorométhane par exemple) ou le tétrahydrofuranne, en présence d'une base organique telle que la triéthylamine, la diméthylaminopyridine, à une température voisine de 20°C.

L'étape e s'effectue par toute méthode connue de l'homme de l'art permettant de passer d'un acide ou un dérivé réactif de cet acide à un carboxamide sans toucher au reste de la molécule et notamment les méthodes préférées décrites précédemment.

L'étape f s'effectue généralement au sein d'un solvant inerte tel que le tétrahydrofuranne, le diméthylformamide, un solvant chloré (chloroforme, dichloroéthane par exemple), un solvant aromatique (benzène, toluène par exemple), à une température comprise entre 10°C et la température d'ébullition du milieu réactionnel.

Les composés de formule (I) pour lesquels R représente un radical CR₁R₂ dans lequel R₁ est un atome d'hydrogène et R₂ représente un radical -CH(R₆)NR₃₁R₃₂ , R₃₁ est un atome d'hydrogène et R₃₂ est un radical alkyle, Ar₁ ou -alk-Ar₁ peuvent être préparés par action d'un halogénure HalR₃₁ sur un composé de formule (I) pour lequel R représente un radical CR₁R₂ dans lequel R₁ est un atome d'hydrogène et R₂ représente un radical -CH(R₆)NR₃₁R₃₂, R₃₁ et R₃₂ sont des atomes d'hydrogène.

Cette réaction s'effectue dans un solvant polaire inerte tel que l'acétonitrile, le tétrahydrofuranne ou le diméthylformamide, en présence d'une base organique ou minérale (carbonate de métalalcalin (sodium, potassium par exemple), trialkylamine (triéthylamine, diméthylaminapyridine par exemple)), à une température comprise entre 0°C et la température d'ébullition du solvant, en présence éventuellement de palladium ou de l'un de ses sels ou complexes.

Les composés de formule (I) pour lesquels R représente un radical CR₁R₂ dans lequel R₁ est un atome d'hydrogène et R₂ représente un radical -CH(R₆)NR₃₁R₃₂, R₃₁ est un atome d'hydrogène et R₃₂ est un radical alkyle peuvent également être préparés par action d'un composé de formule (I) correspondant pour lequel R représente un radical CR₁R₂ dans lequel R₁ est un atome d'hydrogène et R₂ représente un radical - CO-R₆ sur une amine HNR₃₁R₃₂ pour laquelle R₃₁ est un atome d'hydrogène et R₃₂ est un radical alkyle.

Cette réaction s'effectue généralement au sein d'un solvant inerte tel qu'un solvant chloré (dichlorométhane, dichloroéthane par exemple), en présence d'un agent réducteur tel que le triacétoxyborohydrure de sodium, à une température comprise entre 0°C et 70°C.

Les composés de formule (I) pour lesquels R représente un radical CR₁R₂ dans lequel R₁ est un atome d'hydrogène et R₂ représente un radical -CH(R₆)NR₃₁R₃₂, R₃₁ et R₃₂ sont des radicaux alkyle, Ar₁ ou -alk-Ar₁ peuvent être préparés par action d'un halogénure HalR₃₂ sur un composé de formule (I) pour lequel R représente un radical CR₁R₂ dans lequel R₁ est un atome d'hydrogène et R₂ représente un radical -CH(R₆)NR₃₁R₃₂, R₃₁ est un atome d'hydrogène et R₃₂ est un radical alkyle, Ar₁ ou -alk-Ar₁.

Cette réaction s'effectue dans un solvant polaire inerte tel que l'acétonitrile, le tétrahydrofuranne ou le diméthylformamide, en présence d'une base organique ou minérale (carbonate de métalalcalin (sodium, potassium par exemple), trialkylamine (triéthylamine, diméthylaminapyridine par exemple)), à une température comprise entre 0°C et la température d'ébullition du solvant, en présence éventuellement de palladium ou de l'un de ses sels ou complexes.

Les composés de formule (I) pour lesquels R représente un radical CR₁R₂ dans lequel R₁ est un atome d'hydrogène et R₂ représente un radical -CH(R₆)NR₃₁R₃₂, R₃₁ est un atome d'hydrogène et R₃₂ est un radical alkyle (2-6C) ou -alk(2-6C)-Ar₁ peuvent être préparés par action d'un aldéhyde RaCHO pour lequel Ra est un radical alkyle ou -alk-Ar₁ sur un composé de formule (I) pour lequel R représente un radical CR₁R₂ dans lequel R₁ est un atome d'hydrogène et R₂ représente un radical -CH(R₆)NR₃₁R₃₂, R₃₁ et R₃₂ sont des atomes d'hydrogène.

Cette réaction s'effectue dans un solvant inerte, tel que le dichlorométhane, le dichloroéthane, le toluène ou le tétrahydrofuranne, à une température comprise entre 0°C et 50°C, en présence d'un agent réducteur tel que le triacétoxyborohydrure de sodium ou le cyanoborohydrure de sodium.

Les composés de formule (I) pour lesquels R représente un radical CR₁R₂ dans lequel R₁ est un atome d'hydrogène et R₂ représente un radical -CH(R₆)NR₃₁R₃₂, R₃₁ est un radical alkyle, Ar₁ ou -alk-Ar₁ et R₃₂ est un radical alkyle (2-6C)ou -alk(2-6C)-Ar₁ peuvent être préparés par action d'un aldéhyde RaCHO pour lequel Ra est un radical alkyle ou -alk-Ar₁ sur un composé de formule (I) pour lequel R représente un radical CR₁R₂ dans lequel R₁ est un atome d'hydrogène et R₂ représente un radical -CH(R₆)NR₃₁R₃₂, R₃₁ est un atome d'hydrogène et R₃₂ est un radical alkyle, Ar₁ ou -alk-Ar₁.

Cette réaction s'effectue dans un solvant inerte, tel que le dichlorométhane, le dichloroéthane, le toluène ou le tétrahydrofuranne, à une température comprise entre 0°C et 50°C, en présence d'un agent réducteur tel que le triacétoxyborohydrure de sodium ou le cyanoborohydrure de sodium.

Les composés de formule (I) pour lesquels R représente un radical CR₁R₂ dans lequel R₁ est un atome d'hydrogène et R₂ représente un radical -CH(R₆)NR₃₁R₃₂, R₃₁ et R₃₂ forment avec l'atome d'azote auquel ils sont rattachés un hétérocycle choisi parmi aziridinyle, azétidinyle, pyrrolidinyle, pipéridinyle peuvent être préparés par action d'un dihalogénure Hal-alk(2-5C)-Hal sur un composé de formule (I) pour lequel R représente un radical CR₁R₂ dans lequel R₁ est un atome d'hydrogène et R₂ représente un radical -CH(R₆)NR₃₁R₃₂, R₃₁ et R₃₂ sont des atomes d'hydrogène.

Cette réaction s'effectue dans un solvant polaire inerte tel que l'acétonitrile, le tétrahydrofuranne ou le diméthylformamide, en présence d'une base organique ou minérale (carbonate de métalalcalin (sodium, potassium par exemple), trialkylamine (triéthylamine, diméthylaminapyridine par exemple)), à une température comprise entre 0°C et la température d'ébullition du solvant, en présence éventuellement de palladium ou de l'un de ses sels ou complexes.

Les composés de formule (I) pour lesquels R représente un radical CR₁R₂ dans lequel R₁ est un atome d'hydrogène et R₂ représente un radical -CH₂-COR₆, -CH₂-CH(R₆)-NR₃₁R₃₂, -CH₂-C(=NOalk)R₆ peuvent être préparés selon le schéma réactionnel suivant :

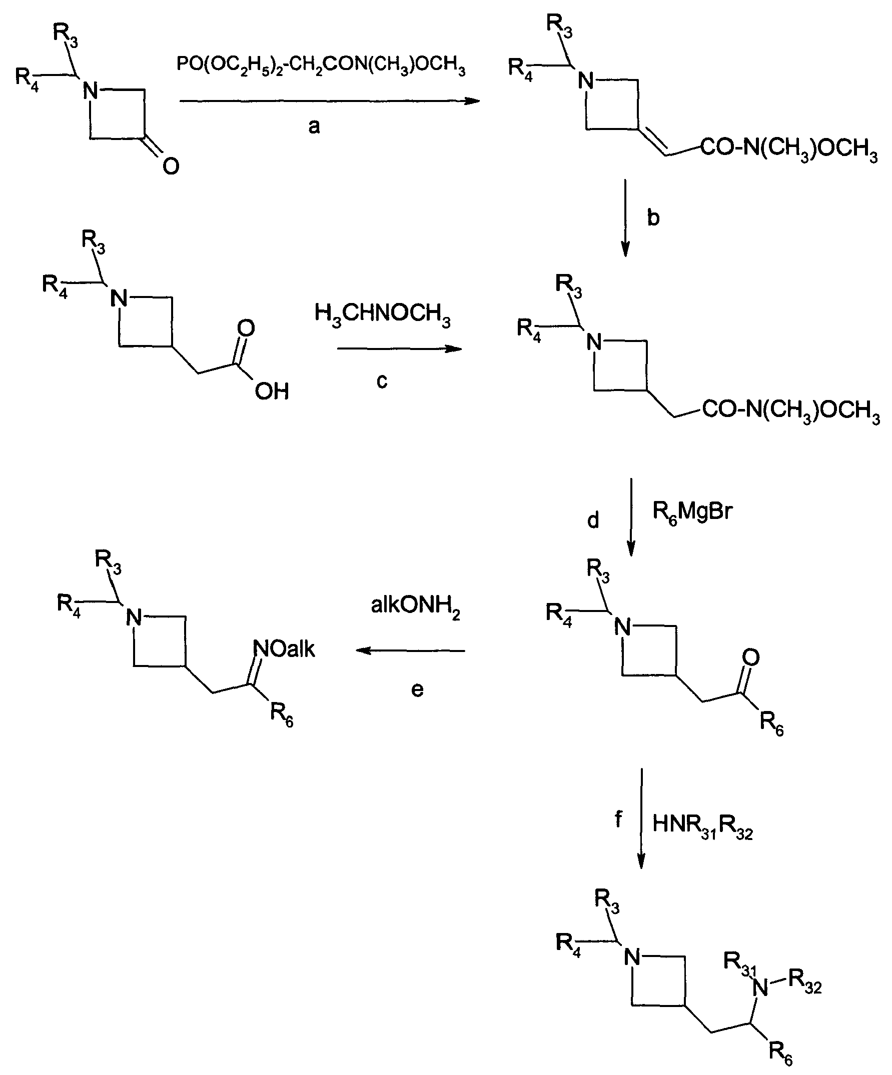

Dans ces formules R₃, R₄, R₆, R₃₁, R₃₂ ont les mêmes significations que dans la formule (I) et alk représente un radical alkyle.

L'étape a s'effectue généralement au sein d'un solvant tel que le tétrahydrofuranne, à une température comprise entre 20°C et la température d'ébullition du milieu réactionnel.

L'étape b s'effectue généralement au sein d'un solvant inerte tel qu'un alcool aliphatiqe (méthanol par exemple), un solvant chloré (chloroforme, dichlorométhane) ou un mélange de ces solvants, en présence d'un agent réducteur tel que NaBH₄, à une température comprise entre 0°C et la température d'ébullition du milieu réactionnel.

L'étape c s'effectue par toute méthode connue de l'homme de l'art permettant de passer d'un acide ou un dérivé réactif de cet acide à un carboxamide sans toucher au reste de la molécule et notamment les méthodes préférées décrites précédemment.

L'étape d s'effectue généralement au sein d'un solvant inerte tel qu'un éther comme le tétrahydrofuranne, à une température voisine de 0°C. Les organomagnésiens sont préparés selon les méthodes connues de l'homme de l'art telles que celles décrites dans les exemples.

L'étape e s'effectue généralement au sein d'un solvant inerte tel qu'un alcool aliphatique 1-4C tel que le méthanol, en présence d'acétate de sodium, à une température comprise entre 20°C et la température d'ébullition du milieu réactionnel.

L'étape f s'effectue au sein d'un solvant inerte tel qu'un solvant chloré (dichlorométhane, dichloroéthane par exemple), en présence d'un agent réducteur tel que le triacétoxyborohydrure de sodium, à une température comprise entre 0°C et 70°C.

Les composés de formule (I) pour lesquels R représente un radical CR₁R₂ dans lequel R₁ représente un radical cyano, -S-alk-NR₁₆R₁₇, -NHR₁₅, alkyle ou -NR₂₀R₂₁ et R₂ représente un radical -C(R₈)(R₁₁)(R₁₂) dans lequel R₈ est un atome d'hydrogène peuvent être préparés selon le schéma réactionnel suivant :

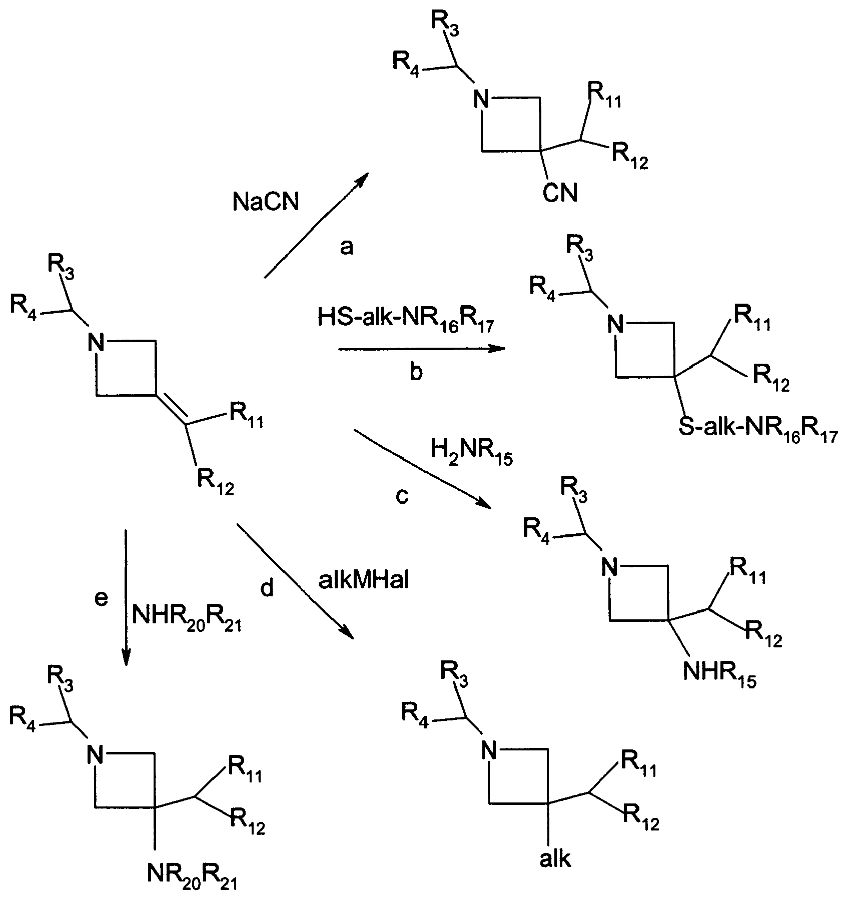

Dans ces formules R₃, R₄, R₁₁, R₁₂, R₁₅, R₁₆ et R₁₇ ont les mêmes significations que dans la formule (I), alk représente un radical alkyle, Hal représente un atome d'halogène et M représente un métal et de préférence le cuivre.

L'étape a s'effectue de préférence au sein d'un solvant polaire tel que le diméthylsulfoxyde, à une température comprise entre 20 et 50°C.

L'étape b s'effectue de préférence au sein d'un solvant inerte tel que le diméthylsulfoxyde, le tétrahydrofuranne, l'acétonitrile, en présence d'une base telle qu'un carbonate de métal alcalin (carbonate de potassium par exemple) ou l'hydroxyde d'ammonium, à une température comprise entre 20°C et la température d'ébullition du milieu réactionnel.

L'étape c s'effectue de préférence au sein d'un solvant inerte tel que le diméthylsulfoxyde, le tétrahydrofuranne, l'acétonitrile, en présence d'une base telle qu'un carbonate de métal alcalin (carbonate de potassium par exemple) ou l'hydroxyde d'ammonium, à une température comprise entre 20°C et la température d'ébullition du milieu réactionnel.

L"étape d s'effectue de préférence au sein d'un solvant inerte tel qu'un éther (éther éthylique), tétrahydrofuranne à une température comprise entre -78°C et 20°C .

L'étape e s'effectue de préférence au sein d'un solvant inerte tel que le diméthylsulfoxyde, le tétrahydrofuranne, l'acétonitrile, le dichlorométhane, le dichloroéthane, en présence d'une base telle qu'un carbonate de métam alcalin (carbonete de potassium par exemple) ou l'hydroxyde d'ammonium, à une température comprise entre 20°C et la température d'ébullition du milieu réactionnel.

Les composés de formule (I) pour lesquels R représente un radical CR₁R₂ dans lequel R₁ représente un radical -alk-NR₁₈R₁₉, R₁₈ et R₁₉ représentent un atome d'hydrogène peuvent être préparés par réduction du composé de formule (I) correspondant pour lequel R représente un radical CR₁R₂ dans lequel R₁ représente un radical cyano.

Cette réaction s'effectue généralement au sein d'un solvant inerte tel que le tétrahydrofuranne, l'éther éthylique, le toluène, à une température comprise entre 0°C et la température d'ébullition du milieu réactionnel, en présence d'un agent réducteur tel que l'hydrure d'aluminium.

Les composés de formule (I) pour lesquels R représente un radical CR₁R₂ dans lequel R₁ représente un radical -alk-NR₁₈R₁₉, R₁₈ représente un atome d'hydrogène et R₁₉ représente un radical alkyle, cycloalkyle, cycloalkylalkyle, cycloalkylcarbonyle, - SO₂alk, -CO-NHalk ou -COOalk peuvent être préparés par action d'un halogénure HalR₁₉, Hal représente un halogène sur un composé de formule (I) pour lequel R représente un radical CR₁R₂ dans lequel R₁ représente un radical -alk-NR₁₈R₁₉, R₁₈ et

R₁₉ représentent un atome d'hydrogène.

Cette réaction s'effectue dans un solvant polaire inerte tel que l'acétonitrile, le tétrahydrofuranne ou le diméthylformamide, en présence d'une base organique ou minérale (carbonate de métalalcalin (sodium, potassium par exemple), trialkylamine (triéthylamine, diméthylaminapyridine par exemple)), à une température comprise entre 0°C et la température d'ébullition du solvant, en présence éventuellement de palladium ou de l'un de ses sels ou complexes.

Les composés de formule (I) pour lesquels R représente un radical CR₁R₂ dans lequel R₁ représente un radical -alk-NR₁₈R₁₉, R₁₈ représente un radical alkyle et R₁₉ représente un radical alkyle, cycloalkyle, cycloalkylalkyle, cycloalkylcarbonyle, - SO₂alk, -CO-NHalk ou -COOalk peuvent être préparés par action d'un halogénure d'alkyle sur un composé de formule (I) pour lequel R représente un radical CR₁R₂ dans lequel R₁ représente un radical -alk-NR₁₈R₁₉, R₁₈ représente un atome d'hydrogène et R₁₉ représente un radical alkyle, cycloalkyle, cycloalkylalkyle, cycloalkylcarbonyle, -SO₂alk, -CO-NHalk ou -COOalk.

Cette réaction s'effectue dans un solvant polaire inerte tel que l'acétonitrile, le tétrahydrofuranne ou le diméthylformamide, en présence d'une base organique ou minérale (carbonate de métalalcalin (sodium, potassium par exemple), trialkylamine (triéthylamine, diméthylaminapyridine par exemple)), à une température comprise entre 0°C et la température d'ébullition du solvant, en présence éventuellement de palladium ou de l'un de ses sels ou complexes.

Les composés de formule (I) pour lesquels R représente un radical CR₁R₂ dans lequel soit R₁ représente un atome d'hydrogène et R₂ représente un radical -C(R₈)(R₉)(R₁₀) ou -C(R₈)(R₁₁)(R₁₂), soit R₁ représente un radical alkyle, NH-R₁₅, cyano, -S-alk-NR₁₆R₁₇, -alk-NR₁₈R₁₉, ou -NR₂₀R₂₁ et R₂ représente un radical -C(R₈)(R₁₁)(R₁₂) et R₈ représente un radical alkyle peuvent être préparés par alkylation d'un composé de formule (I) correspondant pour lequel R₈ est un atome d'hydrogène.

Cette réaction s'effectue de préférence au moyen d'une base telle qu'un hydrure de métal alcalin (hydrure de sodium par exemple), un amidure de métal alcalin (amidure de sodium par exemple) ou un dérivé organométallique, au sein d'un solvant inerte tel qu'un éther aliphétique (éther éthylique) ou le tétrahydrofuranne, à une température comprise entre -78°C et 30°C, au moyen d'un agent d'alkylation tel qu'un halogènure d'alkyle ou un sulfonate d'alkyle.

Les composés de formule (I) pour lesquels R représente un radical C=C(R₇)SO₂alk peuvent également être préparés selon le schéma réactionnel suivant :

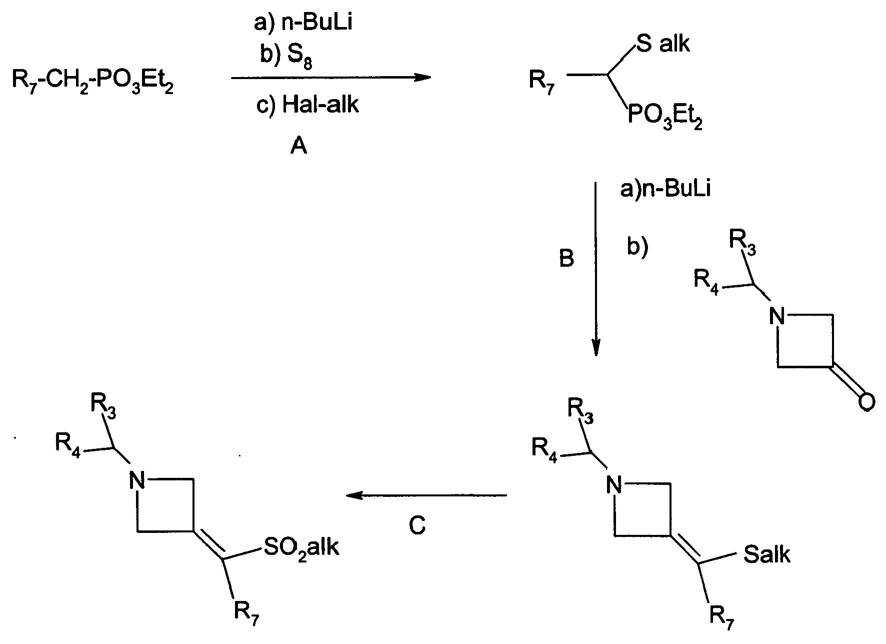

Dans ces formules R₃, R₄ et R₇ ont les mêmes significations que dans la formule (I), alk représente un radical alkyle et Hal représente un atome d'halogène

La réaction A s'effectue généralement au sein d'un solvant inerte tel qu'un éther (éther éthylique par exemple), en présence d'une base forte telle que le tert-butyllithium ou le n-butyllithium, à une température comprise entre -70°C et -50°C, puis addition de soufre puis d'un halogénure d'alkyle (iodure, bromure par exemple).

La réaction B s'effectue généralement au sein d'un solvant inerte tel qu'un éther (tétrahydrofuranne par exemple), en présence d'une base forte telle que le tert-butyllithium ou le n-butyllithium, à une température comprise entre -70°C et -50°C, puis addition de l'azétidin-3-one, retour à température ambiante et hydrolyse.

La réaction C s'effectue par toutes méthodes connues permettant d'oxyder un dérivé soufré sans toucher au reste de la molécule comme celles décrites précédemment.

Les composés de formule (I) pour lesquels R représente un radical CHR₃₃ et R₃₃ représente un radical -NHCOR₃₄ peuvent être préparés selon le schéma réactionnel suivant :

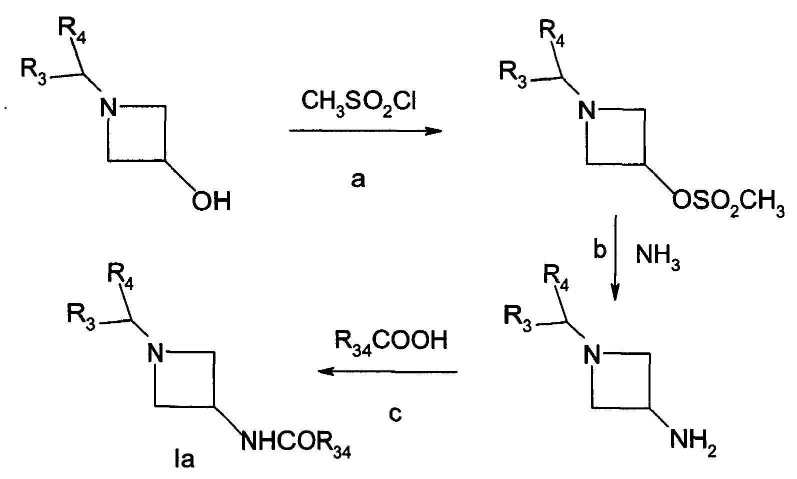

Dans ces formules R₃, R₄ et R₃₄ ont les mêmes significations que dans la formule (I).

L'étape a s'effectue généralement au sein d'un solvant inerte tel que le tétrahydrofuranne, le dioxanne, un solvant chloré (dichlorométhane, chloroforme par exemple), à une température comprise entre 15°C et 30°C, en présence d'une base telle qu'une trialkyamine (triéthylamine, dipropyléthylamine par exemple) ou au sein de la pyridine, à une température comprise entre 0°C et 30°C,.

L'étape b s'effectue de préférence, au sein du méthanol, en autoclave, à une température comprise entre 50 et 70°C.

L'étape c s'effectue généralement en présence d'un agent de condensation utilisé en chimie peptidique tel qu'un carbodiimide (par exemple le 1-(3-diméthylaminopropyl)-3-éthylcarbodiimide, le N,N'-dicyclohexylcarbodiimide) ou le

N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température d'ébullition du mélange réactionnel. On peut également utiliser un dérivé réactif de l'acide comme un chlorure d'acide, éventuellement en présence d'un accepteur d'acide tel qu'une base organique azotée (trialkylamine, pyridine, diaza-1,8 bicyclo[5.4.0]undécène-7 ou diaza-1,5 bicyclo[4.3.0]nonène-5 par exemple), dans un solvant tel que cité ci-dessus, ou un mélange de ces solvants, à une température comprise entre 0°C et la température d'ébullition du mélange réactionnel.

Les dérivés R₃₄COOH sont commerciaux ou peuvent être obtenus selon les méthodes décrites dans R.C. LAROCK, Comprehensive Organic Transformations, VCH editor.

Les composés de formule (I) pour lesquels R représente un radical CHR₃₃ et R₃₃ représente un radical -N(R₃₅)-Y-R₃₆ peuvent être préparés selon le schéma réactionnel suivant : 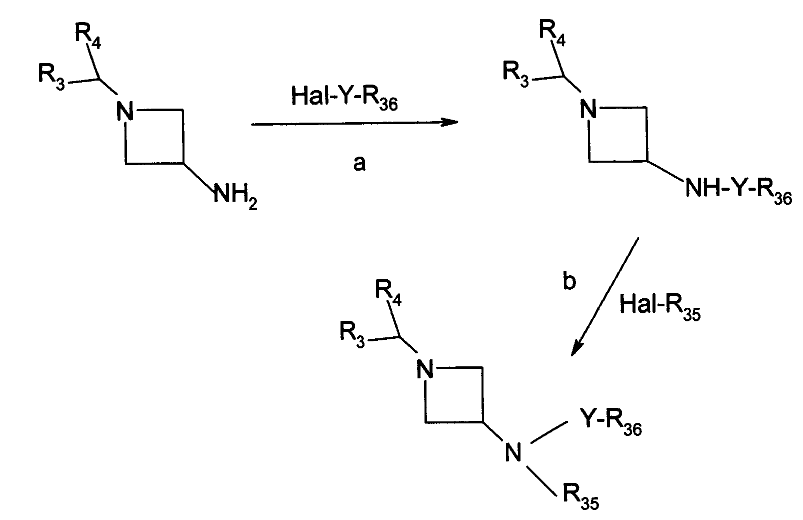 dans ces formules Y, R₄, R₃ et R₃₆ R₃₅ ont les mêmes significations que dans la formule (I), Hal représente un atome d'halogène et, de préférence, un atome d'iode, de chlore ou de brome;

L'étape a s'effectue généralement au sein d'un solvant inerte tel que le tétrahydrofuranne, le dioxanne, un solvant chloré (dichlorométhane, chloroforme par exemple), en présence d'une amine telle qu'une trialkylamine (triéthylamine par exemple), à une température comprise entre 5°C et 20°C.

L'étape b s'effectue généralement au sein d'un solvant inerte tel que le tétrahydrofuranne, en présence d'hydrure de sodium, à une température 0°C et la température d'ébullition du milieu réactionnel.

Les dérivés Hal-SO₂R₃₆ sont commercialisés ou peuvent être obtenus par halogénation des acides sulfoniques correspondants, notamment in situ en présence de chlorosulfonylisocyanate et d'alcool, au sein d'un solvant halogéné (dichlorométhane, chloroforme par exemple).

Les dérivés Hal-CO-R₃₆ sont commerciaux ou peuvent être préparés selon les méthodes décrites dans R.C. LAROCK, Comprehensive Organic Transformations, VCH editor.

Les composés de formule (I) peuvent également être préparés selon le schéma réactionnel suivant :

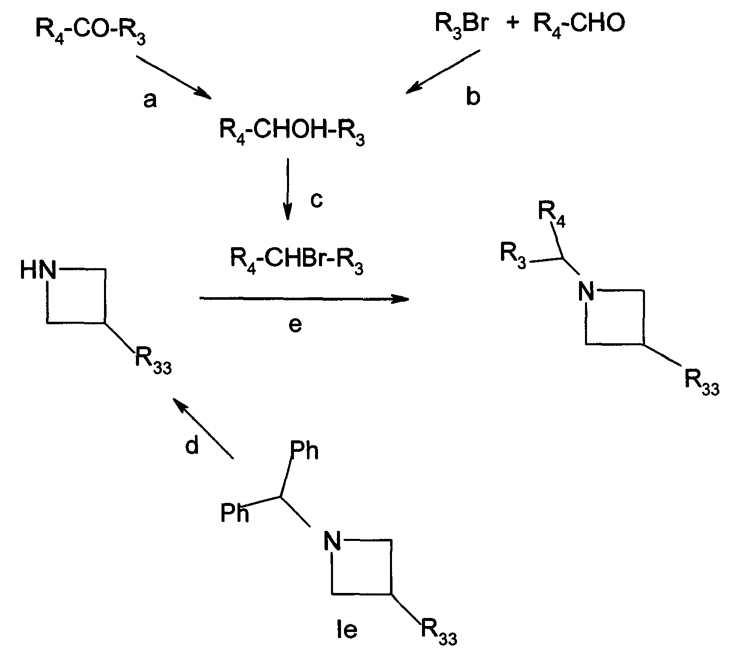

Dans ces formules R₃₃, R₄ et R₃ ont les mêmes significations que dans la formule (I) et Ph représente un phényle.

L'étape a s'effectue généralement au sein d'un alcool tel que le méthanol, en présence de borohydrure de sodium, à une température voisine de 20°C.

Dans l'étape b, on prépare le magnésien du dérivé bromé et le fait réagir, au sein d'un solvant inerte tel que l'éther éthylique ou le tétrahydrofuranne, à une température comprise entre 0°C et la température d'ébullition du milieu réactionnel.

L'étape c s'effectue au moyen d'un agent d'halogénation tel que l'acide bromhydrique, le bromure de thionyle, le chlorure de thionyle, un mélange de triphénylphosphine et de tétrabromure ou tétrachlorure de carbone, au sein de l'acide acétique ou un solvant inerte tel que le dichlorométhane, le chloroforme, le tétrachlorure de carbone ou le toluène, à une température comprise entre 0°C et la température d'ébullition du milieu réactionnel.

L'étape d s'effectue au moyen d'hydrogène, en présence de charbon palladié, au sein d'un alcool tel que le méthanol, à une température voisine de 20°C.

L'étape e s'effectue au sein d'un solvant inerte tel que l'acétonitrile, en présence d'un carbonate de métal alcalin (carbonate de potassium par exemple), et d'iodure de potassium, à une température comprise entre 20°C et la température d'ébullition du milieu réactionnel.

Les dérivés R₃Br et les dérivés R₄-CHO sont commercialisés ou peuvent être obtenus selon les méthodes décrites par exemple par R.C. LAROCK, Comprehensive Organic Transformations, VCH editor.

Les composés de formule (I) pour lesquels R représente un radical CHR₃₃ et R₃₃ représente un radical -N(R₃₅)-Y-R₃₆ dans lequel R₃₆ est un radical phényle substitué par hydroxy peuvent également être préparés par hydrolyse d'un composé de formule (I) correspondant pour lequel R₃₃ représente un radical -N(R₃₅)-Y-R₃₆ dans lequel R₃₆ est un radical phényle substitué par alcoxy.

Cette hydrolyse s'effectue généralement au sein d'un solvant inerte tel qu'un solvant chloré (dichlorométhane, chloroforme par exemple), au moyen de tribromure de bore, à une température voisine de 20°C.

Les composés de formule (I) pour lesquels R représente un radical CHR₃₃ et R₃₃ représente un radical -N(R₃₅)-Y-R₃₆ dans lequel R₃₆ est un radical phényle substitué par hydroxyalkyle(1C) peuvent également être préparés par action de l'hydrure de diisobutylaluminium sur un composé de formule (I) correspondant pour lequel R₃₃ représente un radical -N(R₃₅)-Y-R₃₆ dans lequel R₃₆ est un radical phényle substitué par alcoxycarbonyle.

Cette réaction s'effectue généralement au sein d'un solvant inerte tel que le toluène, au moyen d'hydrure de diisopropylaluminium, à une température comprise entre - 50°C et 25°C.

Les composés de formule (I) pour lesquels R représente un radical CHR₃₃ et R₃₃ représente un radical -N(R₃₅)-Y-R₃₆ dans lequel R₃₆ est un radical phényle substitué par pyrrolidinyl-1 peuvent également être préparés par action de pyrrolidine et d'un composé de formule (I) correspondant pour lequel R₃₃ représente un radical -N(R₃₅)-Y-R₃₆ dans lequel R₃₆ est un radical phényle substitué par fluor.

Cette réaction s'effectue de préférence au sein d'un solvant inerte tel que le diméthylsulfoxyde, à une température de 90°C.

Les composés de formule (I) pour lesquels R représente un radical CHR₄₆ et R₄₆ représente un radical -N(R₄₇)R₄₈ dans lequel R₄₈ est un atome d'hydrogène, -N(R₄₇)-CO-R₄₈, -N(R₄₇)-SO₂R₄₉, R₄₇ est un radical -C(R₅₄)(R₅₅)-Ar₃ ou -C(R₅₄)(R₅₅)-Het₃ et R₅₅ est un atome d'hydrogène peuvent être préparés selon le schéma réactionnel suivant :

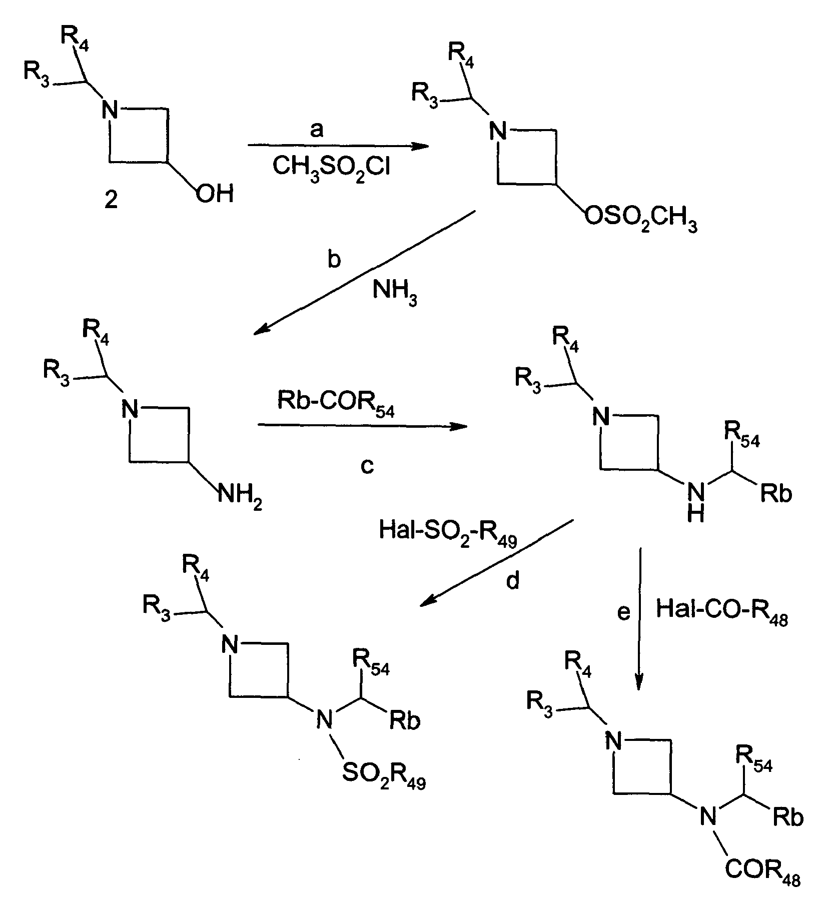

Dans ces formules R₄, R₃, R₄₉ et R₅₄ ont les mêmes significations que dans la formule (I), Rb représente radical Ar₃ ou Het₃, Ar₃ et Het₃ ayant les mêmes significations que dans la formule (I) et Hal représente un atome d'halogène et de préférence chlore ou brome.

L'étape a s'effectue généralement au sein d'un solvant inerte tel que le tétrahydrofuranne, le dioxanne, un solvant chloré (dichlorométhane, chloroforme par exemple), à une température comprise entre 15 et 30°C, en présence d'une base telle qu'une trialkylamine (triéthylamine, dipropyléthylamine par exemple) ou au sein de la pyridine, à une température entre 0 et 30°C

L'étape b s'effectue de préférence au sein du méthanol, en autoclave, à une température comprise entre 50 et 70°C.

L'étape c s'effectue généralement au sein d'un solvant inerte tel qu'un solvant chloré (dichlorométhane par exemple), en présence de triacétoxyborohydrure de sodium et d'acide acétique, à une température voisine de 20°C

Les étapes d et e s'effectuent généralement au sein d'un solvant inerte tel que le tétrahydrofuranne, le dioxanne, un solvant chloré (dichlorométhane, chloroforme par exemple), en présence d'une amine telle qu'une trialkylamine (triéthylamine par exemple), à une température comprise entre 5°C et 20°C.

Les dérivés Rb-COR₅₄ sont commercialisés ou peuvent être obtenus selon les méthodes décrites par exemple par R.C. LAROCK, Coprehensive Organic Transformations, VCH editor.

Les dérivés Hal-SO₂R₄₉ sont commercialisés ou peuvent être obtenus par halogénation des acides sulfoniques correspondants, notamment in situ en présence de chlorosulfonylisocyanate et d'alcool, au sein d'un solvant halogéné (dichlorométhane, chloroforme par exemple).

Les dérivés Hal-COR₄₈ sont commercialisés ou peuvent être préparés par halogénation des acides carboxyliques correspondants, notamment in situ en présence de chlorure de thionyle au sein d'un solvant halogéné (dichlorométhane, chloroforme par exemple).

Les composés de formule (I) pour lesquels R représente un radical CHR₄₆ et R₄₆ représente un radical -N(R₄₇)R₄₈ peuvent être préparés selon le schéma réactionnel suivant :

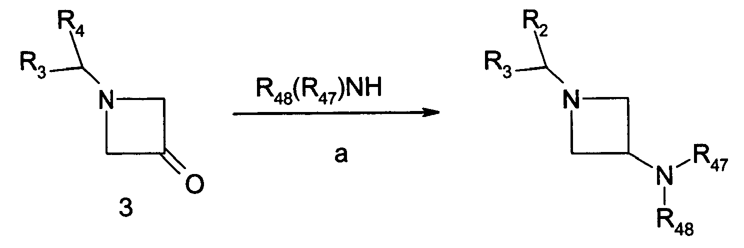

Dans ces formules R₄, R₃, R₄₇ et R₄₈ ont les mêmes significations que dans la formule (I).

Cette réaction s'effectue généralement au sein d'un solvant inerte tel qu'un solvant chloré (dichlorométhane par exemple), en présence de triacétoxyborohydrure de sodium et d'acide acétique, à une température voisine de 20°C.

Les composés HN(R₄₇)R₄₈ sont commercialisés ou peuvent être préparés selon les méthodes classiques connues de l'homme de l'art ou par application ou adaptation des méthodes décrites par Park K.K. et coll., J. Org. Chem., 60 (19) 6202 (1995); Kalir A. Et coll., J. Med. Chem., 12 (3) 473 (1969); Sarges R., J. Org. Chem., 40 (9) 1216 (1975); Zaugg H.E., J. Org. Chem., 33 (5) 2167 (1968); Med. Chem., 10, 128 (1967); J. Am. Chem. Soc., 2244 (1955); Chem. Ber., 106, 2890 (1973); Chem. Pharm. Bull., 16 (10) 1953 (1968); Bull. Soc. Chim. Fr., 835 (1962).

Les azétidinones 3 peuvent être obtenus par oxydation des azétidinoles 2 correspondants, de préférence au sein de diméthylsulfoxyde, au moyen du complexe trioxyde de soufre-pyridine, à une température voisine de 20°C ou au moyen de diméthylsulfoxyde, en présence de chlorure d'oxalyle et de triéthylamine, à une température comprise entre -70°C et -50°C.

Les composés de formule (I) pour lesquels R représente un radical CHR₄₆ et R₄₆ représente un radical -N(R₄₇)COR₄₈ ou -N(R₄₇)SO₂R₄₉ peuvent être préparés selon le schéma réactionnel suivant :

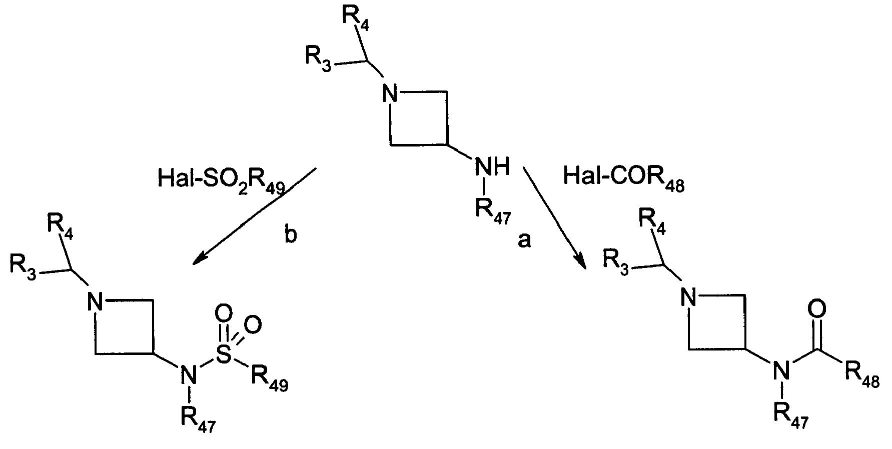

Dans ces formules, R₄, R₃, R₄₇, R₄₈ et R₄₉ ont les mêmes significations que dans la formule (I) et Hal représente un atome d'halogène et de préférence chlore.

Les étapes a et b s'effectuent généralement au sein d'un solvant inerte tel que le tétrahydrofuranne, le dioxanne, un solvant chloré (dichlorométhane, chloroforme par exemple), en présence d'une amine telle qu'une trialkylamine (triéthylamine par exemple), à une température comprise entre 5°C et 20°C.

Les composés de formule (I) pour lesquels R représente un radical CHR₄₆ et R₄₆ représente un radical -N(R₄₇)-SO₂-R₄₉ pour lequel R₄₇ est un radical Het₃ ou Ar₃ peuvent être préparés selon le schéma réactionnel suivant :

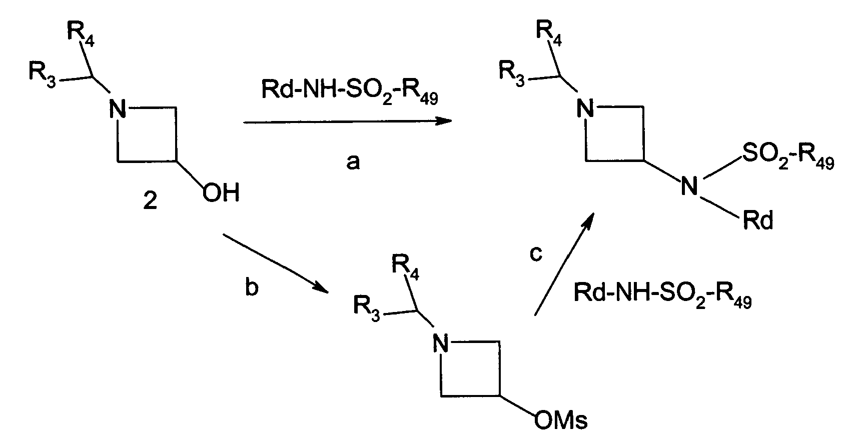

Dans ces formules R₄, R₃ et R₄₉ ont les mêmes significations que dans la formule (I), Rd représente un radical Ar₃ ou Het₃ (Het₃ et Ar₃ ayant les mêmes significations que dans la formule (I)) et Ms représente un radical méthylsulfonyloxy.

L'étape a s'effectue généralement au sein d'un solvant inerte tel que le tétrahydrofuranne, en présence de triphénylphosphine et de diéthylazodicarboxylate, à une température comprise entre 0°C et la température d'ébullition du milieu réactionnel.

L'étape b s'effectue généralement au sein d'un solvant inerte tel que le tétrahydrofuranne, le dioxanne, un solvant chloré (dichlorométhane, chloroforme par exemple), à une température comprise entre 15°C et 30°C, en présence d'une base telle qu'une trialkyamine (triéthylamine, dipropyléthylamine par exemple) ou au sein de la pyridine, à une température entre 0°C et 30°C.

L'étape c s'effectue de préférence, au sein d'un solvant inerte tel que le dioxanne, en présence de CsCO₃, au reflux du mélange réactionnel.

Les dérivés pour lesquels Rd représente un hétérocycle azoté N-oxydé peuvent être réduits en composé non oxydé selon la méthode décrite par SANGHANEL E. Et coll., Synthesis 1375 (1996).

Les dérivés Rd-NH-SO₂R₄₉ peuvent être obtenus selon le schéma réactionnel suivant

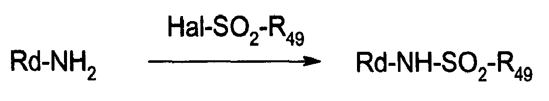

Dans ces formules Hal représente un atome d'halogène et Rd représente un radical Het₃ ou Ar₃. La réaction s'effectue au sein d'un solvant inerte tel que le tétrahydrofuranne, le dioxanne, un solvant chloré (dichlorométhane, chloroforme par exemple), à une température comprise entre 15°C et 30°C, en présence d'une base telle qu'une trialkyamine (triéthylamine, dipropyléthylamine par exemple) ou au sein de la pyridine, à une température comprise entre 0°C et 30°C.

Les dérivés pour lesquels Rd représente un hétérocycle azoté N-oxydé peuvent être obtenus selon la méthode décrite par RHIE R., Heterocycles, 41 (2) 323 (1995).

Les composés de formule (I) pour lesquels R représente un radical CHR₄₆ et R₄₆ représente un radical -N(R₄₇)-SO₂-R₄₉ pour lequel R₄₇ est un radical pipérid-4-yle éventuellement substitué sur l'azote par un radical alkyle peuvent également être préparés selon le schéma réactionnel suivant :

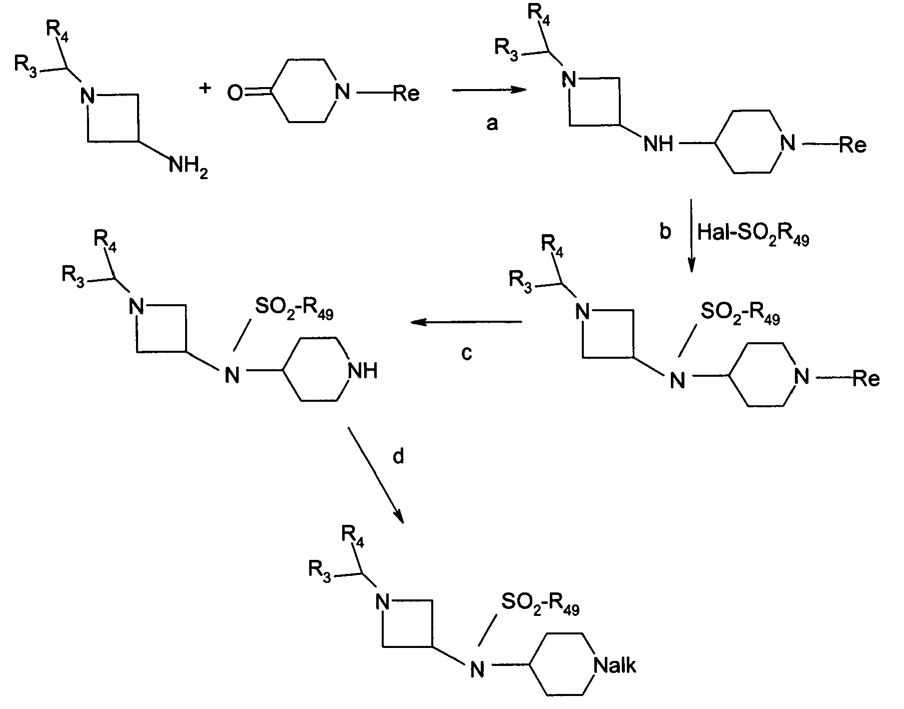

Dans ces formules R₄, R₃ et R₄₉ ont les mêmes significations que dans la formule (I), alk représente un radical alkyle et Re représente un radical tert-butylcarbonyloxy.

L'étape a s'effectue au sein d'un solvant inerte tel qu'un solvant chloré (dichlorométhane par exemple), en présence d'un hydrure tel que le triacétoxyborohydrure de sodium et d'acide acétique, à une température comprise entre 0°C et la température d'ébullition du milieu réactionnel.

L'étape b s'effectue généralement au sein d'un solvant inerte tel que le tétrahydrofuranne, le dioxanne, un solvant chloré (dichlorométhane, chloroforme par exemple), en présence d'une amine telle qu'une trialkylamine (triéthylamine par exemple), à une température comprise entre 5°C et 20°C.

L'étape c s'effectue au moyen d'acide chlorhydrique, au sein du dioxanne, à une température comprise entre 0°C et la température d'ébullition du milieu réactionnel.

L'étape d s'effectue par tout moyen connu de l'homme de l'art pour alkyler une amine sans toucher au reste de la molécule. On peut par exemple utiliser un halogènure d'alkyle, en présence d'une base organique telle que la triéthylamine, un hydroxyde de métal alcalin (soude, potasse par exemple), éventuellement en présence de bromure de tétrabutylammonium, au sein d'un solvant inerte tel que le diméthylsulfoxyde, le diméthylformamide ou la pyridine, à une température comprise entre 20 et 50°C.

Les composés de formule (I) pour lesquels R représente un radical CHR₄₆ et R₄₆ représente un radical -N(R₄₇)-SO₂-R₄₉ pour lequel R₄₇ est un radical phényle substitué par un radical pyrrolid-1-yle peuvent également être préparés par action de pyrrolidine sur un composé de formule (I) correspondant pour lequel R₄₆ représente un radical -N(R₄₇)SO₂R₄₉ pour lequel R₄₇ est un radical phényle substitué par un atome d'halogène.

Cette réaction s'effectue de préférence, au sein du diméthylsulfoxyde, à une température comprise entre 50 et 95°C.

Il est entendu pour l'homme du métier que, pour la mise en oeuvre des procédés selon l'invention décrits précédemment, il peut être nécessaire d'introduire des groupes protecteurs des fonctions amino, hydroxy et carboxy afin d'éviter des réactions secondaires. Ces groupes sont ceux qui permettent d'être éliminés sans toucher au reste de la molécule. Comme exemples de groupes protecteurs de la fonction amino on peut citer les carbamates de tert-butyle ou de méthyle qui peuvent être régénérées au moyen d'iodotriméthylsilane ou d'allyle au moyen de catalyseurs du palladium. Comme exemples de groupes protecteurs de la fonction hydroxy, on peut citer les triéthylsilyle, tert-butyldiméthylsilyle qui peuvent être régénérés au moyen de fluorure de tétrabutylammonium ou bien les acétals dissymétriques (méthoxyméthyle, tétrahydropyranyle par exemple) avec régénération au moyen d'acide chlorhydrique. Comme groupes protecteurs des fonctions carboxy, on peut citer les esters (allyle, benzyle par exemple), les oxazoles et les 2-alkyl-1,3-oxazolines. D'autres groupes protecteurs utilisables sont décrits par GREENE T.W. et coll., Protecting.Groups in Organic Synthesis, second edition, 1991, Jonh Wiley & Sons.

Les composés de formule (I) peuvent être purifiés par les méthodes connues habituelles, par exemple par cristallisation, chromatographie ou extraction.

Les énantiomères des composés de formule (I) peuvent être obtenus par dédoublement des racémiques par exemple par chromatographie sur colonne chirale selon PIRCKLE W.H. et coll., asymmetric synthesis, vol. 1, Academic Press (1983) ou par formation de sels ou par synthèse à partir des précurseurs chiraux. Les diastéréoisomères peuvent être préparés selon les méthodes classiques connues (cristallisation, chromatographie ou à partir des précurseurs chiraux).

Comme exemples de sels pharmaceutiquement acceptables, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-β-oxynaphtoate, nitrate, oxalate, pamoate, phosphate, salicylate, succinate, sulfate, tartrate, théophyllinacétate et p-toluènesulfonate.

### Exemple1 :

Le N-{1-[bis-(4-chlorophényl)méthyl]azétidin-3-yl}-N-pyrid-3-yl-méthylsulfonamide peut être préparé en opérant de la façon suivante : A une solution de 0,144 g de N-{1-[bis-(4-chlorophényl)méthyl]azétidin-3-yl}-N-(1-oxyde-pyrid-3-yl)-méthylsulfonamide dans 5 cm³ de chloroforme, on coule 0,042 cm³ de trichlorure de phosphore, puis chauffe le mélange à la température du reflux. Après 1 heure et 30 minutes d'agitation, le mélange réactionnel est laissé revenir à température ordinaire, puis est additionné de 5 cm³ d'acide chlorhydrique 0,1N, puis agité et décanté. La phase organique est diluée avec 20 cm³ de chloroforme, séchée sur sulfate de magnésium, filtrée, puis concentrée à sec sous pression réduite (2,7 kPa). Le résidu est chromatographié sur une colonne de gel de silice (granulométrie 0,063-0,200 mm, hauteur 9 cm, diamètre 1,8 cm), en éluant sous une pression de 0,1 bar d'argon avec un mélange de dichlorométhane et de méthanol (95/5 en volumes) et en recueillant des fractions de 15 cm³. Les fractions 2 à 4 sont réunies et concentrées à sec sous pression réduite (2,7 kPa). Le résidu est agité avec 15 cm³ d'oxyde de diéthyle, la suspension est filtrée, le solide essoré puis séché sous pression réduite (2,7 kPa). On obtient 35 mg de N-{1-[bis-(4-chlorophényl)méthyl]azétidin-3-yl}-N-pyrid-3-yl-méthylsulfonamide, sous la forme d'un solide crème [Spectre de R.M.N. ¹H (300 MHz, CDCl₃, δ en ppm) : de 2,80 à 2,95 (mt : 2H); 2,87 (s : 3H); 3,51 (t dédoublé, J = 7 et 1,5 Hz : 2H); 4,18 (s : 1H); 4,65 (mt : 1H); de 7,15 à 7,35 (mt : 8H); 7,37 (dd large, J = 8 et 5 Hz : 1H); 7,64 (d démultiplié, J = 8 Hz : 1H); 8 ,52 (d large, J = 2 Hz : 1H); 8,61 (d large, J = 5 Hz : 1H)].

### exemple 2 :

### Méthode 1 :

Le N-{1-[bis-(4-chlorophényl)méthyl]azétidin-3-yl}-N-(3,5-difluorophényl)-méthylsulfonamide peut être préparé en opérant de la façon suivante : A un mélange de 1,23 g de méthylsulfonate de 1-[bis(4-chlorophényl)méthyl]azétidin-3-yle et de 0,66 g de N-(3,5-difluorophényl)méthylsulfonamide, dans 25 cm³ de dioxane, on ajoute 1,0 g de carbonate de césium. Après 5 heures d'agitation à la température du reflux puis 20 heures à 20°C, le mélange réactionnel est additionné de 50 cm³ d'oxyde de diéthyle et de 30 cm³ de saumure, puis est agité et décanté. La phase organique est séchée sur sulfate de magnésium, filtrée puis concentrée à sec à 50°C sous pression réduite (2,7 kPa). L'huile orange obtenue est chromatographiée sur une colonne de gel de silice (granulométrie 0,040-0,063 mm, hauteur 25 cm, diamètre 2,0 cm), en éluant sous une pression de 0,5 bar d'argon avec un mélange de cyclohexane et d'acétate d'éthyle (65/35 en volumes) et en recueillant des fractions de 10 cm³. Les fractions 6 à 10 sont réunies et concentrées à sec sous pression réduite (2,7 Kpa). Le résidu est chromatographié sur une colonne de gel de silice (granulométrie 0,040-0,063 mm, hauteur 15 cm, diamètre 1,0 cm), en éluant sous une pression de 0,5 bar d'argon avec un mélange de cyclohexane et d'acétate d'éthyle (65/35 en volumes) et en recueillant des fractions de 5 cm³. La fraction 7 est concentrée à sec sous pression réduite (2,7 kPa). On obtient 0,11 g de N-{1-[bis-(4-chlorophényl)méthyl]azétidin-3-yl}-N-(3,5-difluorophényl)-méthylsulfonamide, sous la forme d'une poudre blanche [Spectre de R.M.N ¹H (300 MHz, CDCl₃, δ en ppm) : 2,82 (s : 3H); 2,85 (mt : 2H); 3,52 (t dédoublé, J = 7 et 2 Hz : 2H); 4,22 (s : 1H); 4,47 (mt : 1H); de 6,75 à 6,90 (mt : 3H); de 7,20 à 7,35 (mt : 8H)].

### Méthode 2

A une solution de 1,41 g de 1-[bis-(4-chlorophényl)méthyl]azétidin-3-ol et de 0,95 g de N-(3,5-difluorophényl)méthylsulfonamide dans 100 cm³ de tétrahydrofuranne anhydre, on ajoute sous argon 0,78 cm³ d'azodicarboxylate de diéthyle et 1,31 g de triphénylphosphine. Après 16 heures d'agitation à 20°C, 300 cm³ d'acétate d'éthyle sont additionnés, le mélange réactionnel est lavé 2 fois avec 100 cm³ d'eau, séché sur sulfate de magnésium et concentré à sec sous pression réduite (2,7 kPa). Le résidu est chromatographié sur une colonne de gel de silice (granulométrie 0,20-0,063 mm, hauteur 50 cm, diamètre 4 cm), en éluant sous une pression de 0,6 bar d'argon avec un mélange de cyclohexane et d'acétate d'éthyle (75/25 en volumes) et en recueillant des fractions de 125 cm³. Les fractions 6 à 12 sont réunies et concentrées à sec sous pression réduite (2,7 kPa). On obtient 1,8 g d'un solide qui est dissous à chaud dans un mélange acétate d'éthyle/diisopropyle éther (15/2 en volume), refroidi, dilué avec 100 cm³ de pentane pour amorcer la cristallisation. Après filtration et séchage, on obtient 1,0g de N-{1-[bis-(4-chlorophényl)méthyl]azétidin-3-yl}-N-(3,5-difluorophényl)-méthylsulfonamide sous la forme de cristaux blancs fondants à 154°C.

Le N-(3,5-difluorophényl)méthylsulfonamide, peut être préparé en opérant de la façon suivante : A une solution de 3,5 g de 3,5-difluoroaniline dans 75 cm³ de dichlorométhane, on ajoute lentement 2,0 cm³ de chlorure de méthylsulfonyle, 3,8 cm³ de triéthylamine et 20 mg de 4-diméthylaminopyridine. Après 20 heures d'agitation à 20°C, le mélange réactionnel, additionné de 20 cm³ de dichlorométhane et de 20 cm³ d'eau, est agité puis décanté. La phase organique est séchée sur sulfate de magnésium, filtrée, puis concentrée à sec sous pression réduite (2,7 kPa). Le résidu est chromatographié sur une colonne de gel de silice (granulométrie 0,063-0,200 mm, hauteur 20 cm, diamètre 2,0 cm), en éluant sous une pression de 0,1 bar d'argon avec du dichlorométhane et en recueillant des fractions de 25 cm³. Les fractions 14 à 20 sont réunies et concentrées à sec sous pression réduite (2,7 kPa). On obtient 0,66 g de N-(3,5-difluorophényl)méthylsulfonamide, sous la forme d'une poudre blanche.

Le méthylsulfonate de 1-[bis(4-chlorophényl)méthyl]azétidin-3-yle peut être préparé en opérant de la façon suivante : A une solution de 12 g de 1-[bis-(4-chlorophényl)méthyl]azétidin-3-ol dans 200 cm³ de dichlorométhane, on ajoute sous argon en 10 minutes 3,5 cm³ de chlorure de méthylsulfonyle, puis refroidit à +5°C et coule en 10 minutes 3,8 cm³ de pyridine. Après 30 minutes d'agitation à +5°C puis 20 heures à 20°C, le mélange réactionnel est dilué avec 100 cm³ d'eau et 100 cm³ de dichlorométhane. Le mélange, d'abord filtré est décanté. La phase organique est lavée avec de l'eau, puis séchée sur sulfate de magnésium, filtrée et concentrée à sec sous pression réduite (2,7 kPa). L'huile obtenue est chromatographiée sur une colonne de gel de silice (granulométrie 0,063-0,200 mm, hauteur 40 cm, diamètre 3,0 cm), en éluant sous une pression de 0,5 bar d'argon avec un mélange de cyclohexane et d'acétate d'éthyle (70/30 en volumes) et en recueillant des fractions de 100 cm³. Les fractions 4 à 15 sont réunies et concentrées à sec sous pression réduite (2,7 kPa). On obtient 6,8 g de méthylsulfonate de 1-[bis(4-chlorophényl)méthyl]azétidin-3-yle, sous la forme d'une huile jaune.

Le 1-[bis(4-chlorophényl)méthyl]azétidin-3-ol peut être préparé selon le mode opératoire décrit par KATRITZKY A.R. et coll., J. Heterocycl. Chem., 271 (1994), en partant de 35,5 g de chlorhydrate de [bis(4-chlorophényl)méthyl]amine et 11,0 cm³ d'épichlorhydrine. On isole 9,0 g de 1-[bis(4-chlorophényl)méthyl]azétidin-3-ol.

Le chlorhydrate de [bis(4-chlorophényl)méthyl]amine peut être préparé selon la méthode décrite par GRISAR M. et coll., J. Med. Chem., 885 (1973).

L'effet de synergie de l'association de un ou plusieurs produits qui activent la neurotransmission dopaminergique dans le cerveau et de un ou plusieurs antagonistes CB 1 dans le traitement de la maladie de Parkinson a été déterminé dans un modèle d'akinésie induite par la réserpine chez le rat selon le protocole suivant :

Des rats mâles Sprague-Dawley ont été traités avec la réserpine administrée par voie sous-cutanée à la dose de 3 mg/kg (1ml/kg) afin d'induire une akinésie chez l'animal. 18 heures après ce traitement, l'activité locomotrice de ces animaux a été mesurée et enregistrée à l'aide d'un système automatisé (Videotrack, France). La locomotion, exprimée en centimètres, est estimée par une distance moyenne globale parcourue pendant cette période (n= 11-38 rats par groupe). L'analyse statistique est réalisée par une analyse de variance et une comparaison post-hoc (si approprié) à l'aide d'un test de Mann et Whitney ou de Dunnett. Un effet significatif est noté pour p<0.05.

Les tableaux 1 et 2 démontrent l'effet de synergie de l'association.

Le tableau 1 concerne l'administration ip de l'antagoniste CB1 et le tableau 2 concerne l'administration po de l'antagoniste CB1

Les résultats pour l'administration ip de l'antagoniste CB1 (tableau 1) sont exprimés en pourcentage d'augmentation par rapport à l'activité du quinpirole et en pourcentage de diminution par rapport à l'activité d'une très forte dose de lévodopa.

L'association d'un antagoniste du récepteur CB1 et d'un agoniste dopaminergique D2 (quinpirole) est réalisée de la manière suivante:

Le produit antagoniste CB1 (1.5 mg/kg i.p., 2 ml/kg) et le quinpirole (62.5 µg/kg i.p., 1 ml/kg) sont co-administrés 18 heures après l'injection de réserpine. L'enregistrement de l'activité motrice débute 5 minutes après la co-administration des produits et dure 1 heure.

L'association d'un antagoniste du récepteur CB1 et d'une forte dose de lévodopa (modèle de dyskinésie) est réalisée de la manière suivante :

Le produit antagoniste CB1 (3 mg/kg i.p., 2 ml/kg) et la lévodopa (120 mg/kg + bensérazide 50 mg/kg i.p., 5 ml/kg) sont co-administrés. Le bensérazide est un inhibiteur de la dopa-décarboxylase périphérique, ce qui permet à la lévodopa de franchir la barrière hémato-encéphalique avant sa transformation en dopamine. L'enregistrement de l'activité motrice débute 5 minutes après la co-administration et dure 2.5 heures.

**TABLEAU 1**

| Rats réserpinés | Association au quinpirole (62.5 µg/kg ip) | Association à la lévodopa (120 mg/kg ip) |
|---|---|---|
| **Exemple 2** | +139%*** (1.5 mg/kg i.p.) | - 54% *NS* (3 mg/kg i.p.) |
| **Exemple 1** | +96% ** | -20% NS (1.5 mg/kg) (3 mg/kg non testé) |
| SR141716A 1 mg/kg i.p. | +116%*** | -61% * |

SR141716A: N-(piperidin-1-yl)-5(4-chlorophenyl)-1-(2,4-dichlorophenyl)-4-methyl-1H-pyrazole-3-carboxamidehydrochloride
ANOVA + Mann-Whitney: *p<0.05, **p<0.01, ***p<0.001.

Ces résultats selon l'invention démontrent que les antagonistes du récepteur CB1:
- potentialisent significativement les effets d'un agoniste dopaminergique D2 (réduction des symptômes parkinsoniens)
- et réduisent l'hyperactivité induite par une très forte dose de lévodopa (activité anti-dyskinétique)

Les études par voie orale sont effectuée dans un solvant hydrophobe de formulation Labrafil/Labrasol (40/60%, w/w). Ces produits sont administrés (sous un volume de lml/kg) une heure avant l'agoniste dopaminergique. L'enregistrement de l'activité locomotrice débute 5 min après l'injection intra-péritonéale de l'agoniste dopaminergique et dure 1 heure. L'agoniste dopaminergique D1 est le Cl-APB à 0.3 mg/kg. L'agoniste dopaminergique D2 est le quinpirole à 0.1mg.kg.

Les résultats pour l'administration po de l'antagoniste CB1 à trois doses différentes (1, 3 et 10 mg/kg/po) et 1es résultats (tableau 2) sont exprimés en pourcentage d'augmentation par rapport à l'activité du quinpirole et en pourcentage de diminution par rapport à l'activité d'une forte dose de Cl-APB (SKF 82958)

**TABLEAU 2**

| | Dose Mg/kg po | Association au quinpirole (0.1mg/kg ip) | Association au Cl-APB (0.3 mg/kg ip) |
|---|---|---|---|
| **Exemple** 2 | 1 | +55% NS | -16% NS |
| | 3 | +62% * | -61% * |
| | 10 | +97% ** | -62% * |
| **Exemple** 1 | 1 | -1% NS | +22% NS |
| | 3 | +101% * | -21% NS |
| | 10 | +102% * | -53% * |
| SR141716A | 1 | +57% * NS | -32% NS |
| | 3 | +121% ** | -58% * |
| | 10 | +87% ** | -82%** |

SR141716A : N-(piperidin-1-yl-5(4-chlorophenyl)-1-(2,4-dichlorophenyl)-4-methyl-1H-pyrazole-3-carboxamidehydrochloride
ANOVA + Dunnett: *p<0.05, **p<0.01

Ces résultats selon l'invention démontrent que les antagonistes du récepteur CB1 :
- potentialisent significativement les effets d'un agoniste dopaminergique D2 (réduction des symptômes parkinsoniens)
- et réduisent l'hyperactivité induite par une forte dose de type D1 (activité anti-dyskinétique)

Les composés de l'association peuvent être employés par voie orale, parentérale, transdermale ou rectale soit simultanément soit séparément soit de façon étalée dans le temps.

La présente invention concerne également les compositions pharmaceutiques contenant l'association de un ou plusieurs produits qui activent la neurotransmission dans le cerveau et de un ou plusieurs antagonistes du récepteur CB1 tels que définis précédemment avec un véhicule pharmaceutiquement acceptable.

Comme compositions solides pour administration orale, peuvent être utilisés des comprimés, des pilules, des poudres (capsules de gélatine, cachets) ou des granulés. Dans ces compositions, les principes actifs sont mélangés à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

Les compositions pharmaceutiques renfermant l'association telle que définie précédemment contiennent généralement 0.1 à 500 mg de l'antagoniste CB1. La présente invention concerne également la méthode de traitement de la maladie de Parkinson qui consiste à administrer au patient une association ou une composition pharmaceutique renfermant l'association telle que définie précédemment soit simultanément soit séparément soit de manière étalée dans le temps.

Les doses dépendent de l'effet recherché, de la durée du traitement et de la voie d'administration utilisée; elles sont généralement de 0,1 à 500 mg par jour par voie orale pour un adulte de l'antagoniste CB 1.

D'une façon générale, le médecin déterminera la posologie appropriée en fonction de l'âge, du poids et de tous les autres facteurs propres au sujet à traiter.

## Revendications

1. Composition pharmaceutique par voie orale du N-{1-[bis-(4-chlorophényl)méthyl]azétidin-3-yl}-N-(3,5-difluorophényl)-méthylsulfonamide **caractérisée en ce qu'**elle comprend du Labrafil et du Labrasol.

2. Composition pharmaceutique selon la revendication 1 **caractérisée en ce que** le Labrafil et le Labrasol sont dans des proportions 40/60.

3. Utilisation du Labrafil et du Labrasol en tant que solvant hydrophobe pour la préparation d'une composition pharmaceutique par voie orale du N-{1-[bis-(4-chlorophényl)méthyl]azétidin-3-yl}-N-(3,5-difluorophényl)-méthylsulfonamide.
